# EUROPEAN PATENT SPECIFICATION

(11) **EP 2 828 401 B1**
(45) Date of publication and mention of the grant of the patent: **25.10.2017**
(21) Application number: 13712387.3
(22) Date of filing: 19.03.2013
(51) Int. Cl.: C12Q 1/68

(54) **MEANS AND METHODS FOR RESPONSE PREDICTION OF HEPATITIS B TREATMENT**
MITTEL UND VERFAHREN ZUR REAKTIONSVORHERSAGE EINER HEPATITIS-B-BEHANDLUNG
MOYENS ET PROCÉDÉS DE PRÉDICTION DE RÉPONSE D'UN TRAITEMENT CONTRE L'HÉPATITE B

(30) Priority: 19.03.2012 EP 12160199; 26.09.2012 EP 12186188
(43) Date of publication of application: 28.01.2015
(73) Proprietor: Academisch Medisch Centrum, 1105 AZ Amsterdam Zuidoost (NL)
(72) Inventor: REESINK, Hendrik, Willem, 1105 AZ Amsterdam Zuidoost (NL); JANSEN, Louis, 1105 AZ Amsterdam Zuidoost (NL); KOOTSTRA, Neeltje, Akke, 1105 AZ Amsterdam Zuidoost (NL)
(74) Representative: V.O.
(86) International application number: PCT/NL2013/050204
(87) International publication number: WO 2013/141705

(56) References cited:
- LIFENG LIU ET AL: "Effects of variant rs346473 in ARHGAP24 gene on disease progression of HBV infection in han Chinese population", JOURNAL OF HUAZHONG UNIVERSITY OF SCIENCE AND TECHNOLOGY [MEDICAL SCIENCES] ; MEDICAL SCIENCES, HUAZHONG UNIVERSITY OF SCIENCE AND TECHNOLOGY, HEIDELBERG, vol. 31, no. 4, 7 August 2011 (2011-08-07) , pages 482-487, XP019936034, ISSN: 1993-1352, DOI: 10.1007/S11596-011-0477-1
- "Shows that rs7094971 is on Affymetrix SNP-6 array used in XP019936034", , 7 August 2011 (2011-08-07), XP055069847, Retrieved from the Internet: URL:www.affymetrix.com [retrieved on 2013-07-05]
- "Shows that rs218019 is on the Affymetrix SNP-6 array used in XP019936034", , 7 August 2011 (2011-08-07), XP055069852, Retrieved from the Internet: URL:www.affymetrix.com [retrieved on 2013-07-05]
- BRION M ET AL: "New technologies in the genetic approach to sudden cardiac death in the young", FORENSIC SCIENCE INTERNATIONAL, ELSEVIER SCIENTIFIC PUBLISHERS IRELAND LTD, IE, vol. 203, no. 1-3, 15 December 2010 (2010-12-15), pages 15-24, XP027514414, ISSN: 0379-0738, DOI: 10.1016/J.FORSCIINT.2010.07.015 [retrieved on 2010-08-11]
- MILAN J. SONNEVELD ET AL: "Polymorphisms Near IL28B and Serologic Response to Peginterferon in HBeAg-Positive Patients With Chronic Hepatitis B", GASTROENTEROLOGY, vol. 142, no. 3, 1 March 2012 (2012-03-01) , pages 513-520.e1, XP055070037, ISSN: 0016-5085, DOI: 10.1053/j.gastro.2011.11.025
- MELANIE KOLZ ET AL: "Meta-Analysis of 28,141 Individuals Identifies Common Variants within Five New Loci That Influence Uric Acid Concentrations", PLOS GENETICS, vol. 5, no. 6, 5 June 2009 (2009-06-05), page e1000504, XP055070071, DOI: 10.1371/journal.pgen.1000504
- BUSTER E H C J ET AL: "Factors That Predict Response of Patients With Hepatitis B e Antigen@?Positive Chronic Hepatitis B to Peginterferon-Alfa", GASTROENTEROLOGY, ELSEVIER, PHILADELPHIA, PA, vol. 137, no. 6, 1 December 2009 (2009-12-01), pages 2002-2009, XP026927302, ISSN: 0016-5085, DOI: 10.1053/J.GASTRO.2009.08.061 [retrieved on 2009-09-06]
- LOUIS JANSEN ET AL: "Genome wide association study of Chronic Hepatitis B patients identified a variation in the SLC16A9 gene which is associated with HBsAg loss after treatment with peginterferon and adefovi", 63RD ANNUAL MEETING OF THE AMERICAN-ASSOCIATION-FOR-THE-STUDY-OF-LIVE R-DISEASES (AASLD); BOSTON, MA, USA; NOVEMBER 09 -13, 2012, 1 October 2012 (2012-10-01), XP055069962,

## Description

The invention relates to the fields of biology, virology and medicine. In particular, the invention relates to hepatitis B treatment.

Hepatitis B is an infectious inflammatory disease of the liver caused by the hepatitis B virus (HBV). The virus is mainly transmitted by exposure to body fluids, via sexual contact, blood transfusions, renal dialysis, shared use of needles by drug-addicted persons, perinatal infection, et cetera.

HBV is a member of the Hepadnavirus family. The virus is one of the smallest enveloped viruses, with a diameter of about 42 nm. The virus particle (virion) consists of an outer lipid envelope (mainly containing hepatitis B surface antigen, HBsAg) and an icosahedral nucleocapsid core composed of hepatitis B core antigen (HBcAg). The nucleocapsid encloses the viral DNA and a DNA polymerase that has reverse transcriptase activity. The outer envelope contains embedded proteins that are involved in viral binding of, and entry into, susceptible cells.

Acute HBV infection is characterized by liver inflammation, vomiting, and jaundice. Symptoms typically last for a few weeks and then gradually improve while the immune system clears the infection. The probability of full recovery and establishment of protective immunity increases with age: While only 5% adults will suffer from a persistent infection lasting for more than six months, i.e. chronic HBV infection, this rate rises to 70% for young children, and to 95% for newborns infected perinatally.

Prolonged liver inflammation caused by infection with HBV may result in chronic hepatitis B (CHB) with progression to liver fibrosis, cirrhosis and ultimately hepatocellular carcinoma (HCC) and death. This is why HBV has been classified as a class I carcinogen, i.e. an agent carcinogenic to humans, by WHO's International Agency for the Research on Cancer (IARC). Current guidelines therefore recommend treating patients with CHB with active liver inflammation For example the American Association for the Study of Liver Disease (AASLD) guidelines recommend treatment for patients who present with levels of serum HBV DNA over 2,000 IU/mL and/or with elevated alanine aminotransferase (ALT) levels (>2 times upper limit of normal).

CHB can be divided into four phases. In persons who are infected perinataly, an immune tolerant phase occurs most frequently. This phase is characterized by the detection of the hepatitis B "e antigen" (HBeAg). Anti-HBe antibodies are not detectable during this phase. Plasma HBV DNA levels are above 20,000 IU/mL and often much higher.
A second phase is the immune active phase. During this phase ALT values are increased and some patients may loose HBeAg and convert to anti-HBe antibodies. After HBeAg/ antiHBe seroconversion the amount of plasma HBV DNA usually declines to below 20,000 IU/mL. This phase is named the HBeAg negative chronic inactive hepatitis B phase. Subsequently a HBeAg negative chronic active hepatitis B phase may develop, due to the development of HBV pre-core mutants; the plasma HBV DNA levels are again above 20,000 IU/mL and ALT values are increased (R.B. Takkenberg, thesis University of Amsterdam (2011), The Netherlands, chapter 2, ISBN 9789090263045).

Worldwide, around 400 million people have chronic hepatitis B, and approximately 650,000 people die of HBV-related complications every year. Chronic hepatitis B (CHB) is a dynamic process and, as described above, can be present as inactive and active phases. Typical treatment schemes include interferon alpha or nucleotide/ nucleoside analogs inhibiting the viral DNA polymerase. In studies combination therapy with nucleoside/nucleotide analogs with interferon alpha were used. An important improvement was the introduction of pegylated interferon (e.g. Pegasys®), providing prolonged half-life of interferon, into therapy. For review of epidemiology and therapy of HBV infection see J.L. Dienstag N Engl J Med 2008, 359; 1486-1500.

Despite improved therapy, still only a very small portion of patients will clear the infection (loss of HBsAg and HBV-DNA with or without development of anti-HBs antibodies) under treatment, while most others will not. It would, thus, be desirable to be able to predict a patient's response to HBV therapy, in order to adjust a treatment strategy right from the beginning.

Inability to achieve viral immune clearance, with persistent high levels of HBV viremia, has been shown in multiple studies to be associated with poor clinical outcomes, including liver cirrhosis and hepatocellular carcinoma. Evidence demonstrates that individuals who clear HBV infection have a low risk of these adverse outcomes. However, a considerable amount of patients do not achieve viral clearance after therapy and especially with interferon significant side-effects are common. Several favorable markers for viral clearance have been described in CHB (e.g. viral genotype A and B, early HBeAg seroconversion, lower baseline HBsAg). However, considering the suboptimal efficacy of available therapies it is of great practical value to establish more (and preferably stronger) predictors of response (or non-response) before starting anti-viral treatment.

It is an object of the present invention to provide additional markers for a positive or negative HBV treatment outcome.

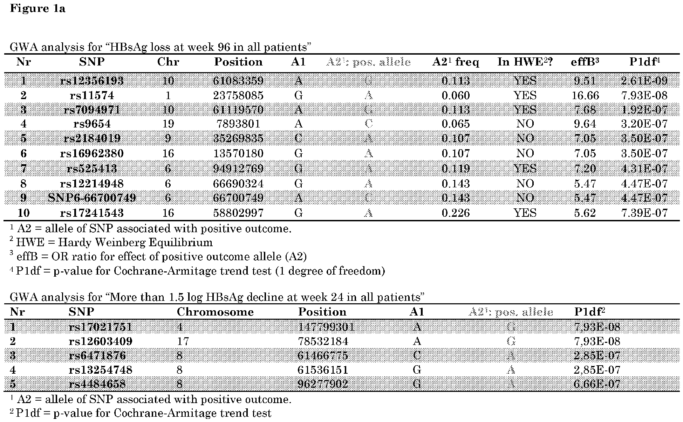

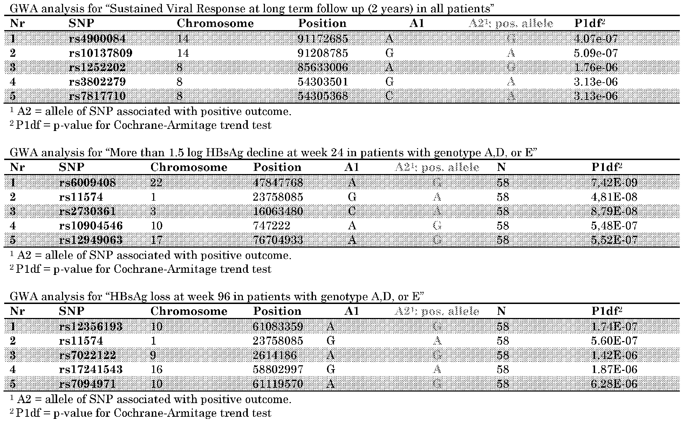

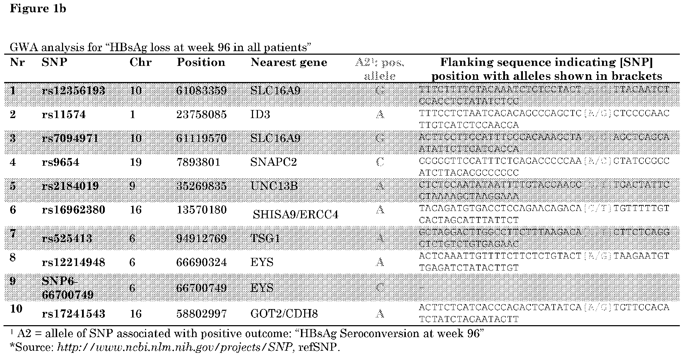

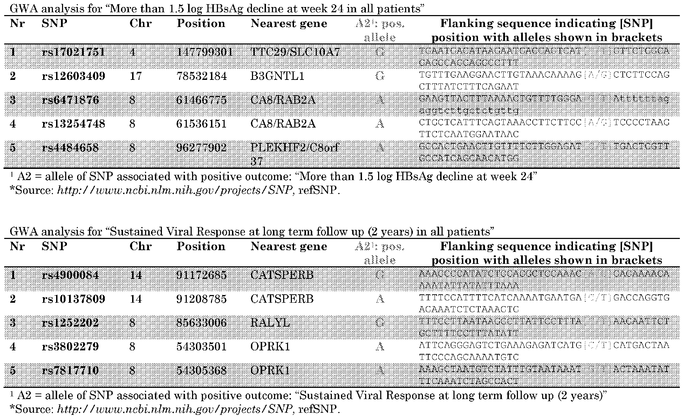

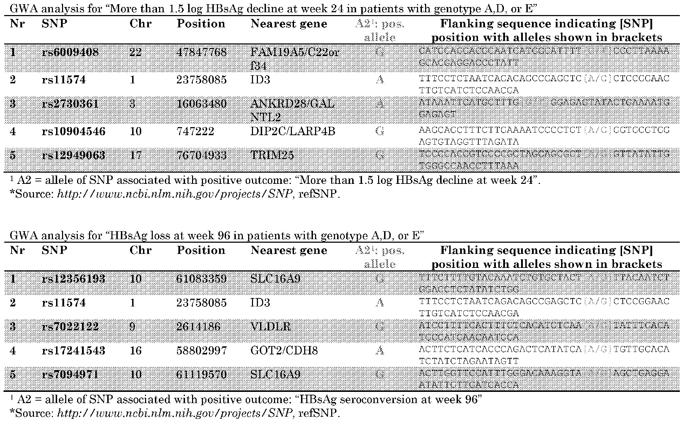

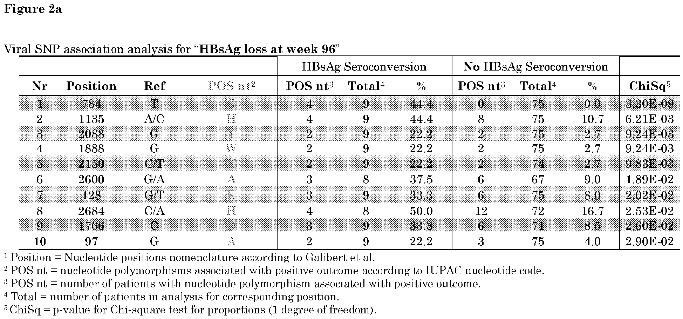

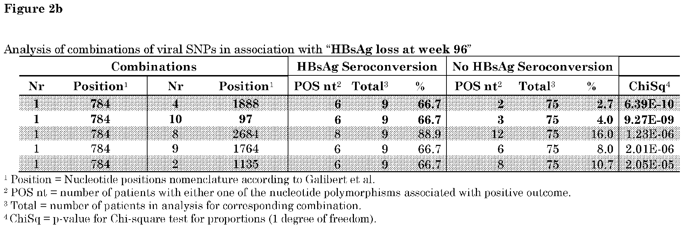

The present invention provides the insight that the outcome of hepatitis B treatment is correlated to nucleotide polymorphisms, both in the genome of the infected individual and in the HBV genome, and to the expression level of carnitine and carnitine derivatives in said individual. Single nucleotide polymorphisms (SNPs) of the human genome associated with HBV treatment outcome are depicted in Figure 1, whereas SNPs of the HBV genome associated with HBV treatment outcome are depicted in Figure 2. Nucleotide positions nomenclature was used according to Galibert et al. (Galibert F, et al. Nature. 1979 Oct 25;281(5733):646-50.). Until the present invention, it was not known that correlations exist between genomic + HBV nucleotide polymorphisms and HBV treatment outcome. It was also unknown that expression levels of carnitine and carnitine derivatives are indicative for HBV treatment outcome. Now that the present invention has provided the insight that nucleotide polymorphisms are associated with treatment outcome, it has become possible to type a nucleic acid-containing sample of an individual by determining at least one of the polymorphisms depicted in Figure 1 and/or 2. Disclosed is a method for typing a nucleic acid-containing sample of a hepatitis B patient, comprising determining an SNP as depicted in Figure 1 and/or Figure 2 in nucleic acid of said sample. Methods for detecting nucleic acid polymorphisms are known in the art and for instance involve nucleic acid extraction, amplification and detection, for instance using common techniques like PCR, MLPA, sequencing, etc. Such well known methods need no further explanation.
It has also become possible to type a sample of a hepatitis B patient by determining the expression level of carnitine and/or a carnitine derivative in said sample. A relatively low level of carnitine and/or a carnitine derivative is indicative for a positive hepatitis B treatment outcome, as outlined in more detail herein below. Disclosed is a method for typing a sample of a hepatitis B patient, comprising determining the expression level of carnitine and/or of a carnitine derivative in said sample. Preferably, the method comprises determining the level of carnitine and/or of a carnitine derivative in said sample.

Carnitine is a quaternary ammonium compound biosynthesized from the amino acids lysine and methionine. Carnitine is involved in fatty acid metabolism, as it transports long-chain acyl groups from fatty acids into the mitochondrial matrix. A carnitine derivative is defined herein as a carnitine-based compound other than carnitine itself, that contains a carnitine moiety, optionally with modifications, substitutions, added groups and/or deletions, which still has at least one fatty acid metabolism property of carnitine. Preferred carnitine derivatives are acetyl-L-carnitine and propionyl-L-carnitine. As used herein, the term "carnitine" embraces L-carnitine and D-carnitine. L-carnitine is, however, preferred.

Methods herein disclosed are particularly suitable for determining whether a certain hepatitis B patient is susceptible to hepatitis B treatment. As used herein, a hepatitis B patient is "susceptible to hepatitis B treatment" if the patient has an improved chance of a positive outcome of hepatitis B treatment, as compared to the mean hepatitis B patient population. A patient with a positive treatment outcome is called a responder. On the other hand, if a patient does not exhibit a positive treatment outcome, such patient is called a non-responder. The invention provides a method for determining whether a hepatitis B patient has an improved chance of a positive outcome of hepatitis B treatment, as compared to the mean hepatitis B patient population, comprising determining whether a nucleic acid-containing sample of said hepatitis B patient comprises the G allele of SNP rs12356193. Methods according to the invention are capable of determining whether a given HBV patient is likely to respond to HBV therapy, before the onset of such therapy. This provides the advantage that patients who will likely not respond can be excluded from therapy, thereby avoiding unnecessary costs, treatment burden and side effects. On the other hand, it may be decided more easily to offer patients with a high chance of a positive outcome HBV therapy, even though the patient's condition may not be optimal for undergoing such therapy, because it is known that his/her chance for a positive treatment outcome is significantly higher than the mean chance.

Sonneveld et al. (2012, Gastroenterology, Vol. 142, No. 3, pp. 513-520) discloses that the presence of two polymorphisms near the IL28B can predict a positive outcome of treatment with Peg-interferon of a hepatitis B patient, provided that adjustment for ethnicity was performed. It fails to teach or suggest detecting the G allele of SNP rs12356193, let alone that this allows for a more robust predictor for anti-HBV treatment success, independent of ethnicity.

As will be understood by those skilled in the art, the aforementioned identification methods are usually not intended to be correct for 100% of the subjects to be analyzed. However, the assessment will be valid for a statistically significant portion of the subjects to be analyzed. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the differentiation will be correct for at least 60%, at least 70%, at least 80%, or at least 90% of the subjects of a given cohort or population.

Figures 1 and 2 provide SNPs of the human and HBV genomes that are associated with HBV treatment outcome. The alleles that are associated with a positive treatment outcome are depicted in the sixth column of Figures 1a and 1b, and in the fourth column of Figure 2a. One aspect of the invention therefore provides a method for determining whether a hepatitis B patient has an improved chance of a positive outcome of hepatitis B treatment, as compared to the mean hepatitis B patient population, comprising determining SNP rs12356193 in combination with at least one other positive outcome-associated allele of an SNP as depicted in Figure 1 or 2. A hepatitis B patient is identified as having an improved chance of a positive outcome of hepatitis B treatment based on the presence of such positive outcome-associated allele. The absence of one or more positive outcome-associated allele(s) of an SNP as depicted in Figure 1 or 2 indicates a lower chance of a positive outcome of hepatitis B treatment.

The term nucleic acid-containing sample relates to a sample of an individual that contains either nucleic acid from said individual, and/or HBV nucleic acid. The term nucleic acid embraces compounds comprising a chain of nucleotides, more preferably DNA and/or RNA. In a preferred embodiment of the invention, said nucleic acid-containing sample is a DNA-containing sample. A saliva swab or blood sample is particularly preferred, since such sample can be taken without much discomfort for the patient or is routinely taken for another purpose. In one embodiment a serum sample is used. An advantage of a serum sample is the fact that such sample is historically more often stored.

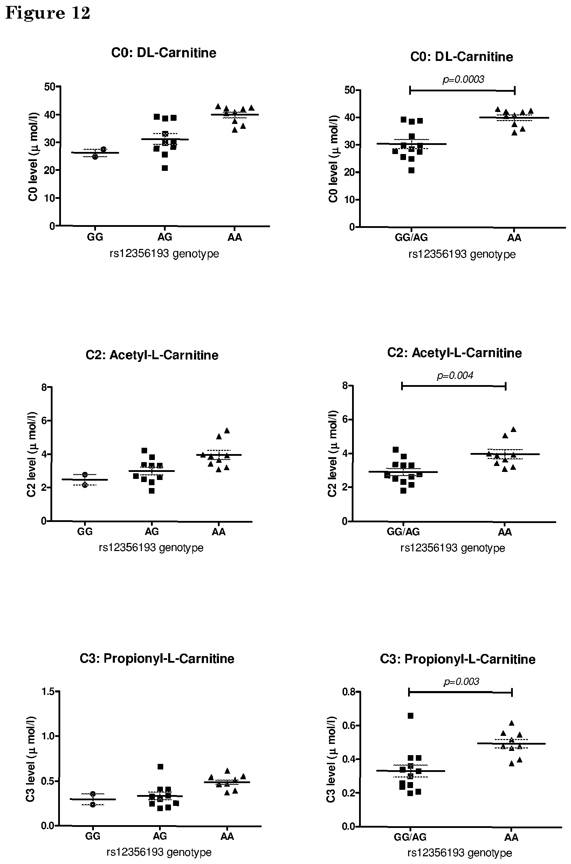

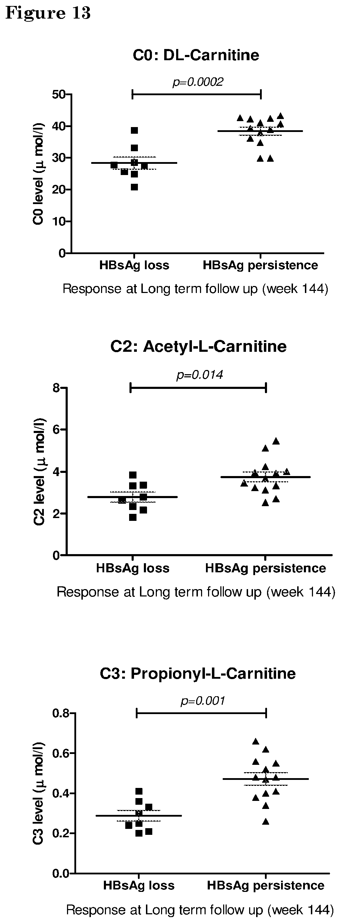

The present disclosure further provides the insight that the (expression) levels of carnitine and carnitine derivatives in a hepatitis B patient susceptible to hepatitis B treatment are significantly lower than the (expression) levels of carnitine and carnitine derivatives in hepatitis B patients who are not susceptible to hepatitis B treatment (see for instance Figures 12 and 13). Now that this insight has been provided, it has become possible to determine whether a certain hepatitis B patient has an improved chance of a positive outcome of hepatitis B treatment, as compared to the mean hepatitis B patient population. For instance, an expression level of carnitine or a carnitine derivative such as acetyl-L-carnitine and proprionyl-L-carnitine of a given patient is determined using any suitable method known in the art. Subsequently, the obtained value is preferably compared with a reference. From said comparison, the tested patient is classified as a responder or a non-responder. In one embodiment, the obtained value from the tested patient is compared with a reference value of a responder group. If the obtained value corresponds to / is essentially equal to this reference value, or is lower, the tested patient is classified as a responder. If, however, the obtained value is significantly higher, the tested patient is classified as a non-responder. Alternatively, or additionally, the obtained value from the tested patient is compared with a reference value of a non-responder group. If the obtained value corresponds to / is essentially equal to this reference value, or is higher, the tested patient is classified as a non-responder. If, however, the obtained value is significantly lower, the tested patient is classified as a responder.

In principle, any sample containing expression products of carnitine and/or carnitine derivatives is suitable for a method herein disclosed. A blood sample or plasma sample may be used, since such sample can be taken without much discomfort for the patient or is routinely taken for another purpose. In one aspect, a serum sample is used. An advantage of a serum sample is the fact that such sample is historically more often stored. Preferably, an "expression level" of carnitine or a carnitine derivative refers to the level of carnitine as such or the carnitine derivative as such in a sample.

Typically, if a plasma sample is used, a hepatitis B patient having a carnitine expression level that is equal to or less than 34 micromole/liter, preferably equal to or less than 33.3 micromole/liter, is classified as a responder, whereas a patient having a carnitine expression level that is more than 34 micromole/liter, preferably more than 33.3 micromole/liter, is classified as a non-responder. A patient having an acetyl-L-carnitine expression level that is equal to or less than 3.89 micromole/liter is classified as a responder, whereas a patient having an acetyl-L-carnitine expression level that is more than 3.89 micromole/liter is classified as a non-responder. A patient having an acetyl-L-carnitine expression level that is equal to or less than 3.0 micromole/liter can be classified as a responder, whereas a patient having an acetyl-L-carnitine expression level that is more than 3.0 micromole/liter is classified as a non-responder. Furthermore, a patient having a proprionyl-L-carnitine expression level that is equal to or less than 0.4 micromole/liter, preferably equal to or less than 0.43 micromole/liter, is typically classified as a responder, whereas a patient having a proprionyl-L-carnitine expression level that is more than 0.4 micromole/liter, preferably more than 0.43 micromole/liter, is classified as a non-responder.

The obtained value from a tested patient may be compared with a reference value of a responder group and with a reference value of a non-responder group, and it is determined which reference value is closest to the obtained test value. The tested patient is then classified as belonging to the group whose reference value is closest to the test value.

Disclosed is therefore a method for determining whether a hepatitis B patient has an improved chance of a positive outcome of hepatitis B treatment, as compared to the mean hepatitis B patient population, further comprising determining whether a sample of said hepatitis B patient comprises an expression level of carnitine or a carnitine derivative that is associated with a positive outcome of hepatitis B treatment. Preferably, it is determined whether a sample of said hepatitis B patient comprises a level of carnitine or a carnitine derivative that is associated with a positive outcome of hepatitis B treatment. Said carnitine derivative may comprise acetyl-L-carnitine and/or proprionyl-L-carnitine. Preferably, the amount of carnitine or carnitine derivative is compared with at least one reference value, as explained above. In one embodiment, it is determined whether the expression level of carnitine in a plasma sample is equal to or less than 33.3 micromole/liter (responder) or more than 33.3 micromole/liter (non-responder). In one embodiment, it is determined whether the expression level of acetyl-L-carnitine in a plasma sample is equal to or less than 3.89 micromole/liter (responder) or more than 3.89 micromole/liter (non-responder). In one embodiment, it is determined whether the expression level of proprionyl-L-carnitine in a plasma sample is equal to or less than 0.43 micromole/liter (responder) or more than 0.43 micromole/liter (non-responder). In a further preferred embodiment, it is determined whether the expression level of carnitine in a plasma sample is equal to or less than 29 micromole/liter (responder) or more than 29 micromole/liter (non-responder). A hepatitis B patient having a carnitine expression level in plasma of 29 micromole/liter or less has an even improved chance of a positive outcome of hepatitis B treatment.

A chronic hepatitis B patient is defined as an individual who has been diagnosed as having a hepatitis B virus infection (also called an HBV infection) for more than six months.
Typically, HBV presence is diagnosed by the detection of at least one viral polypeptide in a sample from a subject, more preferably, at least one of the viral antigens HBs (HBsAg, Genbank Acc. No.: AAL66340.1 GI:18252577, SEQ ID NO: 1), HBc (HBcAg, Genbank Acc. No.: CAA51257.1 GI:288930, SEQ ID NO: 2), and HBe (HBeAg, Genbank Acc. No.: AAM96930.1 GI:22530876, SEQ ID NO: 3) is detected. It is understood by the skilled person that the HBV polypeptides are referenced as biomarkers, not as specific polypeptides, and that the term HBV encompasses various strains of HBV which may comprise sequence variants of the aforementioned HBV polypeptides. Accordingly, the aforementioned polypeptides having the specific sequences deposited under the Genbank accession numbers are to be understood as exemplary sequences representing a biomarker. Hence it is also possible to detect variant polypeptides which vary due to at least one amino acid addition, substitution, deletion and/or modification from the above mentioned HBV polypeptides as long as they are also suitable as biomarkers for an HBV infection as discussed above. Preferably, the variant polypeptides are at least 70%, at least 75%, at least 80%, at least 85%, at least 90%, at least 95%, at least 98% or at least 99% identical to the above mentioned specific polypeptides.

The term "identical" as used herein refers to sequence identity characterized by determining the number of identical nucleotides or amino acids between two nucleic acid sequences or amino acid sequences, respectively, wherein the sequences are aligned so that the highest order match is obtained. It can be calculated using published techniques or methods codified in computer programs such as, for example, BLASTP, BLASTN or FASTA (Altschul 1990, J Mol Biol 215, 403). The percent identity values are, in one aspect, calculated over the entire nucleic acid sequence or amino acid sequence. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Higgins 1989, CABIOS 5, 151) or the programs Gap and BestFit (Needleman 1970, J Mol Biol 48; 443; Smith 1981, Adv Appl Math 2, 482), which are part of the GCG software packet (Genetics Computer Group 1991, 575 Science Drive, Madison, Wisconsin, USA 53711), may be used. The sequence identity values recited herein in percent (%) are to be determined, using the program GAP over the entire sequence region with the following settings: Gap Weight: 50, Length Weight: 3, Average Match: 10,000 and Average Mismatch: 0.000, which, unless otherwise specified, shall always be used as standard settings for sequence alignments.
Also preferably, HBV presence is detected by detecting at least one viral polynucleotide, preferably viral DNA, in a sample from a subject. The nucleotide sequences of the viral polynucleotides or the entire HBV genome are well known in the art. Preferably, HBV nucleotide sequences are deposited under Genbank accession number NC_003977.1. It is understood by the skilled person that the HBV polynucleotides are referenced as biomarkers, not as specific polynucleotides, and that the term HBV encompasses various strains of HBV comprising variant nucleotide sequences. Accordingly, the aforementioned polynucleotides having the specific sequences deposited under the Genbank accession number are to be understood as exemplary sequences representing a biomarker. Hence, it is also possible to detect variant polynucleotides which vary due to at least one nucleotide addition, substitution, deletion and/or modification from the above mentioned polynucleotides as long as they are also suitable as biomarkers for an HBV infection as discussed above. Preferably, the variant polynucleotides are at least 70%, at least 75%, at least 80%, at least 85%, at least 90%, at least 95%, at least 98% or at least 99% identical to the above mentioned specific polynucleotides. Identity can be calculated as set forth herein above.
Preferably, the term HBV infection referred to herein is a chronic HBV infection. A chronic HBV infection is, preferably, characterized by the detectable presence of HBV in a subject for more than six months. More preferably, a chronic HBV infection referred to herein follows the definition published by the Center for Disease Control (CDC), according to which a chronic HBV infection is characterized by the following laboratory criteria: IgM antibodies to hepatitis B core antigen (IgM anti-HBc) negative AND a positive result on one of the following tests: hepatitis B surface antigen (HBsAg), hepatitis B e antigen (HBeAg), or hepatitis B virus (HBV) DNA OR HBsAg positive or HBV DNA positive two times at least 6 months apart (any combination of these tests performed 6 months apart is acceptable).
One preferred embodiment therefore provides a method according to the invention, wherein said hepatitis B patient suffers from chronic hepatitis B.

A nucleic acid sequence at which more than one sequence is possible in a population is referred to herein as a "polymorphic site." Polymorphic sites can allow for differences in sequences based on substitutions, insertions, or deletions. Such substitutions, insertions, or deletions can result in frame shifts, the generation of premature stop codons, the deletion or addition of one or more amino acids encoded by a polynucleotide, alter splice sites, and affect the stability or transport of mRNA. Where a polymorphic site is a single nucleotide in length, the site is referred to as a single nucleotide polymorphism ("SNP").

The term "SNP" refers to a single nucleotide polymorphism at a particular position in the genome of a mammal that varies among a population of individuals. As used herein, an SNP may be identified by its name or by location within a particular sequence. The SNPs identified in Figure 1b are indicated by brackets. For example, the SNP "[A/G]" in the sequence of rs12356193 in Figure 1b indicates that the nucleotide base (or the allele) at that position in the sequence may be either adenine or guanine. The allele associated with a positive outcome of hepatitis B treatment (e.g., a guanine in rs12356193) is indicated in column six of Figures 1a and 1b, or column four of Figure 2a. The nucleotides flanking the SNPs in Figure 1b are the flanking sequences which are useful for identifying the location of the SNP in the genome. As used herein, the nucleotide sequences referred to in the present application encompass the complements of said nucleotide sequences. In addition, as used herein, the term "SNP" encompasses any allele among a set of alleles.

The term "allele" refers to a specific nucleotide among a selection of nucleotides defining an SNP.

The term "positive outcome-associated allele" or "positive outcome allele" refers to an allele that is associated with a positive outcome of hepatitis B treatment. Preferred kinds of hepatitis B treatment and preferred kinds of positive treatment outcome are outlined herein below. The sixth columns of Figures 1a and 1b and the fourth column of Figure 2a show positive outcome-associated alleles according to the present invention.

The term "haplotype" refers to a combination of particular alleles from two or more SNPs.

The term "positive outcome haplotype" refers to a haplotype that is associated with a positive outcome of hepatitis B treatment.

The term "polynucleotide" refers to polymeric forms of nucleotides of any length. The polynucleotides may contain deoxyribonucleotides, ribonucleotides, and/or their analogs. Polynucleotides may have any three-dimensional structure including single-stranded, double-stranded and triple helical molecular structures, and may perform any function, known or unknown. The following are non-limiting embodiments of polynucleotides: a gene or gene fragment, exons, introns, mRNA, tRNA, rRNA, short interfering nucleic acid molecules (siNA), ribozymes, cDNA, recombinant polynucleotides, branched polynucleotides, plasmids, vectors, isolated DNA of any sequence, isolated RNA of any sequence, nucleic acid probes, and primers. A polynucleotide may also comprise modified nucleic acid molecules, such as methylated nucleic acid molecules and nucleic acid molecule analogs.

A "substantially isolated" or "isolated" polynucleotide is one that is substantially free of the sequences with which it is associated in nature. By substantially free is meant at least 50%, at least 70%, at least 80%, or at least 90% free of the materials with which it is associated in nature. An "isolated polynucleotide" also includes recombinant polynucleotides, which, by virtue of origin or manipulation: (1) are not associated with all or a portion of a polynucleotide with which it is associated in nature, or (2) are linked to a polynucleotide other than that to which it is linked in nature, or (3) does not occur in nature.

The term "vector" refers to a DNA molecule that can carry inserted DNA and be perpetuated in a host cell. Vectors are also known as cloning vectors, cloning vehicles or vehicles. The term "vector" includes vectors that function primarily for insertion of a nucleic acid molecule into a cell, replication vectors that function primarily for the replication of nucleic acids, and expression vectors that function for transcription and/or translation of the DNA or RNA. Also included are vectors that provide more than one of the above functions.

With a method according to the present invention, it is possible to determine whether a hepatitis B patient has an improved chance of a positive outcome of hepatitis B treatment. As used herein, a positive outcome can have several clinical manifestations. In one embodiment a positive outcome is defined as a more than 1.5 log HBsAg decline in the blood of said patient. Preferably, said HBsAg decline is observed at or before week 24 of therapy. This means that the amount of detectable HBsAg declines faster than the mean HBsAg decline observed in a hepatitis B population receiving the same kind of treatment. Fast decline is indicative for a higher chance of suppression or cure of hepatitis B, including a higher chance of HBsAg seroconversion although this does not always occur. HBsAg seroconversion is defined as the loss of detectable HBsAg in the blood of a patient and the appearance of anti-HBs antibodies in the blood. One embodiment thus provides a method for determining whether a hepatitis B patient has an improved chance of a more than 1.5 log HBsAg decline in his/her blood at or before week 24 of HBV therapy, as compared to the mean hepatitis B patient population, comprising determining whether a nucleic acid-containing sample of said hepatitis B patient comprises the G allele of SNP rs12356193, optionally in combination with at least one further positive outcome-associated allele of an SNP as depicted in Figure 1 and/or in Figure 2 and/or determining whether a sample of said hepatitis B patient comprises an expression level of carnitine or of a carnitine derivative (preferably acetyl-L-carnitine and/or proprionyl-L-carnitine) that is associated with a positive outcome of hepatitis B treatment.

In a preferred embodiment, a positive outcome of a hepatitis B treatment is defined as a sustained virological response. This means that HBV DNA levels equal to or lower than 2,000 IU/ml blood are obtained and alanine aminotransferase levels are normalized at 2 years of follow up. Further provided is therefore a method for determining whether a hepatitis B patient has an improved chance of a sustained virological response after HBV treatment, as compared to the mean hepatitis B patient population, comprising determining whether a nucleic acid-containing sample of said hepatitis B patient comprises the G allele of SNP rs12356193, optionally in combination with at least one positive outcome-associated allele of an SNP as depicted in Figure 1 and/or Figure 2 and/or determining whether a sample of said hepatitis B patient comprises an expression level of carnitine or of a carnitine derivative (preferably acetyl-L-carnitine and/or proprionyl-L-carnitine) that is associated with a positive outcome of hepatitis B treatment.

In a particularly preferred embodiment, a positive outcome of a hepatitis B treatment is defined as HBsAg loss. This means that HBsAg has disappeared from the blood. HBsAg clearance represents the closest endpoint to a clinical cure, and is associated with improved survival and a lower risk for development of liver cirrhosis and hepatocellular carcinoma. As shown in the Examples, SNPs are disclosed which are even associated with this most favorable outcome. Also, levels of carnitine and carnitine derivatives appear to be correlated with this most favorable outcome. Further provided is therefore a method for determining whether a hepatitis B patient has an improved chance of HBsAg loss after HBV treatment, as compared to the mean hepatitis B patient population, comprising determining whether a nucleic acid-containing sample of said hepatitis B patient comprises the G allele of SNP rs12356193, optionally in combination with at least one positive outcome-associated allele of an SNP as depicted in Figure 1 and/or Figure 2 and/or determining whether a sample of said hepatitis B patient comprises an expression level of carnitine or of a carnitine derivative (preferably acetyl-L-carnitine and/or proprionyl-L-carnitine) that is associated with a positive outcome of hepatitis B treatment. Preferably, said hepatitis B patient has an improved chance of HBsAg loss with anti-HBs seroconversion (meaning that anti-HBs antibodies are detectable in the blood of the individual after treatment).

A preferred embodiment thus provides a method according to the invention for determining whether a hepatitis B patient has an improved chance of a positive outcome of hepatitis B treatment, wherein said positive outcome is selected from the group consisting of HBsAg loss, HBsAg loss with anti-HBs seroconversion, sustained virological response, and a more than 1.5 log HBsAg decline in the blood of said patient at or before week 24 of therapy. Preferably, any of such methods is performed before the start of said hepatitis B therapy, in order to predict treatment outcome.

In one preferred embodiment, the susceptibility of a hepatitis B patient for a positive outcome of hepatitis B treatment is determined with a method according to the invention, wherein said hepatitis B treatment comprises administration of interferon. Preferably, interferon as referred to in this context is an interferon covalently bound to polyethyleneglycol (PEG-interferon). More preferably, the term interferon encompasses interferon 2 alpha, most preferably interferon 2 alpha covalently bound to polyethyleneglycol (PEG-interferon 2 alpha, commercially available as Pegasys®). Other preferred non-limiting examples of currently known therapeutic interferon compounds are PEG-interferon alfa 2b (Peg-Intron), peg-Interferon Lambda, Locteron (which is interferon alpha2b present in biospheres), Consensus interferon (which is a genetically engineered interferon compound sharing 88% homology with IFN-alfa and 30% with IFN-beta) and IFN-gamma.

Treatment of hepatitis B patients with interferon or other immune modulators such as for instance toll like receptor agonists is preferably combined with treatment with an inhibitor of the viral DNA polymerase in order to improve treatment response. Preferably, an inhibitor of the viral DNA polymerase is a nucleotide analogue or nucleoside analogue. Thus, a preferred method according to the invention determines whether a hepatitis B patient has an improved chance of a positive outcome of a treatment with interferon in combination with a nucleotide analogue or nucleoside analogue. More preferably, said treatment comprises treatment with interferon 2 alpha in combination with a nucleotide analogue or nucleoside analogue and even more preferably said treatment comprises treatment with PEG-interferon or PEG-interferon 2 alpha (e.g. pegasys®) or other immune modulators in combination with a nucleotide analogue or nucleoside analogue. Several nucleotide or nucleoside analogues are known in the art. Preferred non-limiting examples are lamivudine, adefovir, adefovir dipivoxil, entecavir, tenofovir, tenofovir disoproxil fumarate and telbivudine. These nucleotide/nucleoside analogues are well known in the art. Shortly, lamivudine is an analogue of cytidine. It can inhibit the reverse transcriptase of hepatitis B. It is phosphorylated to active metabolites that compete for incorporation into viral DNA.
Adefovir is an oral acyclic phosphonate nucleotide analogue which inhibits HBV polymerase by chain termination. The main benefit of adefovir over lamivudine (the first NRTI approved for the treatment of hepatitis B) is that it takes a much longer period of time before the virus develops resistance to it. Adefovir dipivoxil contains two pivaloyloxymethyl units, making it a prodrug form of adefovir.
Entecavir is an oral cyclopentyl guanosine analogue that inhibits HBV DNA priming. Transcription of the negative-stranded HBV DNA and synthesis of the positive-strand HBV DNA is reversed.
Tenofovir is a nucleotide analogue which blocks reverse transcriptase. Tenofovir disoproxil fumarate is a prodrug form of tenofovir.
Telbivudine is the L-isomer of thymidine and causes HBV DNA chain termination.
A particularly preferred embodiment thus provides a method for determining whether a hepatitis B patient has an improved chance of a positive outcome of hepatitis B treatment, as compared to the mean hepatitis B patient population, comprising determining whether a nucleic acid-containing sample of said hepatitis B patient comprises the G allele of SNP rs12356193, optionally in combination with at least one positive outcome-associated allele of an SNP as depicted in Figure 1 and/or Figure 2 and/or whether a sample of said hepatitis B patient comprises an expression level of carnitine or of a carnitine derivative that is associated with a positive outcome of hepatitis B treatment, wherein said hepatitis B treatment comprises administration of interferon (preferably interferon 2 alpha or pegylated interferon, more preferably PEG-interferon 2 alpha) in combination with a nucleoside or nucleotide analogue selected from the group consisting of lamivudine, adefovir, adefovir dipivoxil, entecavir, tenofovir, tenofovir disoproxil fumarate and telbivudine.

In a particularly preferred embodiment, the susceptibility of a hepatitis B patient for a positive outcome of hepatitis B treatment is determined with a method according to the invention, wherein said hepatitis B treatment comprises administration of interferon (preferably interferon 2 alpha or pegylated interferon, more preferably PEG-interferon 2 alpha) in combination with adefovir or tenofovir. Most preferably, said treatment comprises treatment with PEG-interferon 2 alpha, e.g. PEGasys®, in combination with adefovir or tenofovir, preferably adefovir. Usually, the PEGasys® treatment is carried out by subcutaneous administration (weekly subcutaneous injection) for a duration of 48 weeks whereas the viral DNA polymerase inhibitors (nucleoside/nucleotide analoga) are administrated orally for a longer time, i.e. for more than one year in 80% of the patients. In current combination therapy regimes however, the viral DNA polymerase inhibitors are stopped together with PEG-interferon after 48 weeks. For details see also J.L. Dienstag N Engl J Med 2008, 359; 1486-1500.

Figure 1 shows several SNPs of the human genome which are associated with the outcome of HBV treatment. One particularly preferred positive outcome-associated allele is the G allele of SNP rs12356193. As shown in the Examples, this allele of rs12356193 was significantly associated with HBsAg loss at the end of the follow up of HBV treatment, with a p-value of 2.61E-09 and an overall minor allele frequency of 0.11. The invention therefore provides a method for determining whether a hepatitis B patient has an improved chance of a positive outcome of hepatitis B treatment as compared to the mean hepatitis B patient population, comprising determining whether a nucleic acid-containing sample of said hepatitis B patient comprises at least the G allele of SNP rs12356193.

Another particularly preferred positive outcome-associated allele is T784G in the HBV genome, meaning that a guanine is present at HBV position 784 instead of a thymine. As used herein, HBV nucleotide positions are defined as the positions in genotype specific reference sequences adw2 (X02763), aad (D00330), (AB033556), ayw (X02496) and (X75657) for genotypes A, B, C, D and E, respectively, or positions corresponding thereto in other HBV strains. For each individual HBV strain, the skilled person is well capable of determining the nucleotide positions corresponding to the positions of the above mentioned reference strains, for instance by aligning using ClustalW Multiple Alignment in BioEdit sequence analysis software (Hall, T.A. 1999. BioEdit: a user-friendly biological sequence alignment editor and analysis program for Windows 95/98/NT. Nucl. Acids. Symp. Ser. 41:95-98). It is shown in the Examples that the T784G allele is significantly associated with HBsAg loss at week 96 after Bonferroni correction for multiple testing. This allele occurred in 4 out of 9 patients who achieved HBsAg loss at week 96 and in none of the patients who did not achieve HBsAg loss. Disclosed is therefore a method for determining whether a hepatitis B patient has an improved chance of a positive outcome of hepatitis B treatment as compared to the mean hepatitis B patient population, comprising determining whether a nucleic acid-containing sample of said hepatitis B patient comprises at least the HBV T784G allele.
It is advantageous to determine whether more than one positive outcome-associated SNP allele depicted in Figure 1 and/or Figure 2 is present in a nucleic acid-containing sample, in order to increase the predictive value for HBV treatment outcome. A method may comprise determining whether at least 2, preferably at least 3, 4, 5, 6, 7, 8, 9 or 10 positive outcome-associated SNP alleles as depicted in Figure 1 and/or Figure 2 are present in said sample. An HBV patient that comprises at least two positive outcome-associated SNP alleles as depicted in Figure 1 and/or Figure 2 has a so called "positive outcome haplotype".In one preferred embodiment, both SNP rs12356193 and an HBV SNP at position 784 are determined. It is preferably determined whether the G allele of SNP rs12356193 and the HBV T784G allele is present in a nucleic acid-containing sample, preferably in combination with at least one other positive outcome-associated SNP allele as depicted in Figure 1 and/or Figure 2. Most preferably, it is determined whether both the G allele of SNP rs12356193 and the HBV T784G allele are present in a nucleic acid-containing sample, optionally in combination with at least one other positive outcome-associated SNP allele as depicted in Figure 1 and/or Figure 2.

Also disclosed is determining HBV nucleotide polymorphisms at positions 784 and 1888 (or positions corresponding thereto in HBV strains other than the above mentioned consensus strains). The G1888W allele is also associated with HBsAg loss at week 96 after starting HBV treatment, as shown in the Examples. Hence, both testing for HBV nucleotide polymorphisms at positions 784 and 1888 (or positions corresponding thereto in HBV strains other than the above mentioned consensus strains) increases the predictive value for HBV treatment outcome. Of note, the presence of an SNP in the HBV genome is dependent on the HBV genotype, using the genotype specific reference strain as a reference. Therefore, for example the A1135H allele was noted in genotype A, and the C1135H allele in genotypes B, C, D and E.

In another preferred embodiment, HBV SNPs at positions 784, 1888 and/or 97 (or positions corresponding thereto in HBV strains other than the above mentioned consensus strains) are tested. In the Examples it is shown that a G97A allele is also associated with HBsAg loss at week 96 after starting HBV treatment. Hence, testing for HBV nucleotide polymorphisms at positions 784 and 1888 or positions 784 and 97 further increases the predictive value for HBV treatment outcome.

Any of the above mentioned HBV alleles are tested for in combination with at least SNP rs12356193, optionally further in combination with at least one other SNP depicted in Figure 1, in order to increase the predictive value for HBV treatment outcome even further.

In certain embodiments, the invention also provides interferon or another immune modulator, optionally in combination with an inhibitor of the viral DNA polymerase (preferably a nucleoside analogue or nucleotide analogue), for use in a method for treating hepatitis B, characterized in that a hepatitis B patient is treated who has been tested positive for the G allele of SNP rs12356193, optionally in combination with at least one positive outcome-associated SNP allele as depicted in Figure 1 and/or Figure 2 and/or who has been tested positive for a positive outcome-associated level of carnitine or of a carnitine derivative.
As explained above, the presence of such positive outcome-associated SNP allele, and/or the presence of such positive outcome-associated level of carnitine or of a carnitine derivative, in a sample of a given HBV patient is indicative for an increased chance of said individual to be a responder of therapy, as compared to the mean chronic hepatitis B population, which will for instance result in HBsAg loss, sustained virological response and/or a more than 1.5 log HBsAg decline in the blood of said patient at or before week 24 of therapy. Such HBV patient will therefore more easily be prescribed HBV therapy, even though the patient's condition may not be optimal. On the other hand, if a given patient is tested negative for a positive treatment outcome SNP allele or haplotype, and/or negative for a positive treatment outcome-associated level of carnitine or of a carnitine derivative, he/she can be excluded from therapy, thereby avoiding unnecessary costs, treatment burden and side effects. Preferably, a hepatitis B patient is treated with HBV therapy who has been tested positive for at least the G allele of SNP rs12356193 and the T784G allele of the HBV genome, optionally in combination with at least one other positive outcome-related SNP allele as depicted in Figure 1 and/or Figure 2 such as for instance an HBV A/C1135H allele and/or an HBV G1888W allele.
In another preferred embodiment, a hepatitis B patient is treated with HBV therapy who has been tested for a plasma carnitine level and which tested carnitine level was equal to or less than 33.3 micromole/liter, preferably equal to or less than 29 micromole/liter.
In yet another preferred embodiment a hepatitis B patient is treated with HBV therapy who has been tested for a plasma acetyl-L-carnitine level and which tested acetyl-L-carnitine level was equal to or less than 3.89 micromole/liter.
In yet another preferred embodiment a hepatitis B patient is treated with HBV therapy who has been tested for a plasma proprionyl-L-carnitine level and which tested proprionyl-L-carnitine level was equal to or less than 0.43 micromole/liter.

As described herein before, said HBV treatment preferably comprises administration of interferon, preferably interferon 2 alpha or pegylated interferon, more preferably PEG-interferon 2 alpha. Other preferred non-limiting examples of currently known therapeutic interferon compounds are PEG-interferon alfa 2b (Peg-Intron), peg-Interferon Lambda, Locteron, Consensus interferon and IFN-gamma.
Said interferon treatment is preferably combined with an inhibitor of the viral DNA polymerase, such as a nucleoside or nucleotide analogue. Such nucleoside or nucleotide analogue is most preferably selected from the group consisting of lamivudine, adefovir, adefovir dipivoxil, entecavir, tenofovir, tenofovir disoproxil fumarate and telbivudine.

Now that the present invention has provided the insight that the outcome of hepatitis B treatment is associated with certain SNPs in the genome of the infected individual and in the HBV genome, it has become possible to produce a collection of nucleic acid molecules comprising HBV treatment outcome-associated sequences. Such collection is particularly suitable for testing an HBV patient for HBV treatment outcome. Such collection is therefore called a "HBV treatment outcome dedicated collection" or a "HBV dedicated collection". Of course, other nucleic acid sequences may be present in such HBV dedicated collection, such as for instance reference nucleic acids and calibrators, as long as at least 70%, preferably at least 75%, more preferably at least 80%, more preferably at least 85%, more preferably at least 90%, more preferably at least 95% of the polynucleotides of the HBV dedicated collection comprise an SNP that is associated with HBV treatment outcome, preferably selected from the SNPs as depicted in Figure 1 and/or Figure 2. An HBV dedicated collection can for instance be in the form of a kit of parts, an array, microarray or a vector. Further provided is therefore a kit of parts or array or microarray comprising at least two polynucleotides comprising SNP rs12356193, wherein at least 70%, preferably at least 75%, more preferably at least 80%, more preferably at least 85%, more preferably at least 90%, more preferably at least 95% of the polynucleotides comprised by said kit of parts or array or microarray comprise an SNP as depicted in Figure 1 and/or Figure 2.
Such HBV dedicated collection is particularly suitable for testing a nucleic acid-containing sample of an HBV patient in order to assess whether said patient is susceptible to hepatitis B treatment. A use of a kit of parts or array or microarray or vector for typing a nucleic acid-containing sample of a hepatitis B patient is therefore also herewith provided, as well as a use of a kit of parts or array or microarray or vector for determining whether a hepatitis B patient has an improved chance of a positive outcome of hepatitis B treatment, as compared to the mean hepatitis B patient population. As said before, said hepatitis B patient is preferably a chronic hepatitis B patient.

A kit of parts or array or microarray preferably comprises a set of primers or a probe, each of said primers or probe having a length, independently from one another, of between 8 and 50 nucleotides, preferably between 8 and 30 nucleotides, more preferably between 8 and 25 nucleotides, characterized in that at least 70% of said primers or probe are complementary to a sequence comprising an SNP as depicted in Figure 1 or Figure 2. Such primers and probes are particularly suitable for detecting and/or amplifying nucleotide polymorphisms of the human and/or HBV genome that are associated with HBV treatment outcome, thereby allowing typing a nucleic acid-containing sample of a hepatitis B patient. Further disclosed is a use of at least one isolated polynucleotide comprising an SNP as depicted in Figure 1 and/or Figure 2 for typing a nucleic acid-containing sample of a hepatitis B patient. The length of such isolated polynucleotide is preferably between 8 and 50 nucleotides, more preferably between 8 and 30 nucleotides, more preferably between 8 and 25 nucleotides. Preferably, said at least one polynucleotide is at least 80% complementary to a stretch of at least 8, preferably at least 10, more preferably at least 12, more preferably at least 15 nucleotides of any one of the sequences of Figure 1 or 2. Of course, it should be well defined for which allele said polynucleotide is specific. Hence, the nucleotide defining the SNP allele should not vary. The flanking sequences of said SNPs may, however, vary to some extent as compared to the natural flanking sequences. More preferably, at least a polynucleotide is used that is complementary to the G allele of SNP rs12356193 and/or the T784G allele of HBV, since these alleles are significantly associated with a positive HBV treatment outcome.

Also disclosed is a method for assaying for the presence of nucleic acid comprising a positive outcome-associated SNP allele as depicted in Figure 1 and/or Figure 2 in a sample, comprising contacting said sample with at least one polynucleotide comprising an SNP as depicted in Figure 1 and/or Figure 2, and determining whether said polynucleotide hybridizes to sample nucleic acid. Said assay preferably comprises a PCR-based assay or a MLPA-based assay. Preferably, it is determined whether said polynucleotide hybridizes to sample nucleic acid under stringent conditions. The term "hybridizes under stringent conditions" is intended to describe conditions for hybridization and washing under which nucleotide sequences at least 60%, 65%, 70%, 75%, 80%, 85%, 90%, 95%, or 98% identical to each other typically remain hybridized to each other. Such stringent conditions are known to those skilled in the art and can for instance be found in Current Protocols in Molecular Biology, John Wiley & Sons, N.Y (1989), 6.3.1- 6.3.6. A non-limiting example of stringent hybridization conditions are hybridization in 6x sodium chloride/sodium citrate (SSC) at about 45 °C, followed by one or more washes in 0.2. x SSC, 0.1% SDS at 50-65 °C. Again, the nucleotide defining the SNP allele should be well defined. The flanking sequences of the SNP of said at least one polynucleotide is preferably at least 80% complementary to any one of the sequences of Figure 1 or 2. More preferably, a polynucleotide is used wherein the flanking sequences of the SNP are at least 90%, more preferably at least 95%, most preferably completely complementary to any one of the sequences of Figure 1 or 2. At least a polynucleotide is preferably used that is complementary to the G allele of SNP rs12356193 and/or the T784G allele of HBV.
Also disclosed is a kit of parts, array, microarray, vector or use according to the invention, wherein said at least one polynucleotide is capable of amplifying a nucleic acid sequence with a length of between 50 and 600 nucleotides, preferably between 100 and 400 nucleotides, more preferably 150-250 nucleotides, wherein said sequence comprises an SNP as depicted in Figure 1 and/or Figure 2.
Further disclosed is a use of means for determining an expression level of carnitine and/or a carnitine derivative (preferably acetyl-L-carnitine and/or proprionyl-L-carnitine), for typing a sample of a hepatitis B patient.
In certain embodiments, the invention also provides a method for determining whether an individual has a genetic predisposition for an increased chance of a positive outcome of hepatitis B treatment, as compared to the mean hepatitis B patient population, comprising determining whether genomic nucleic acid of said individual comprises at least the G allele of SNP rs12356193, optionally further comprising determining whether a sample of said individual comprises an (expression) level of carnitine and/or a carnitine derivative that is associated with a positive outcome of hepatitis B treatment.

Another application is the specific identification of a gene that is associated with an increased chance of a hepatitis B patient for a positive outcome of hepatitis B treatment, as compared to the mean hepatitis B patient population. This is done by identifying genes involved with the SNPs according to the invention. Once such gene is identified, protein sequences and/or protein expression levels between responders and non-responders are preferably compared, for instance in order to obtain insight into the molecular pathways involved in an increased chance of a positive outcome of HBV treatment. Disclosed is a method for identifying a gene associated with an increased chance of a hepatitis B patient for a positive outcome of hepatitis B treatment, as compared to the mean hepatitis B patient population, comprising:
- identifying a gene containing an SNP as depicted in Figure 1, and
- comparing the expression of said gene in an individual having a positive outcome-associated allele of an SNP as depicted in Figure 1 with the expression of said gene in an individual not having said positive outcome-associated allele for differences indicating that said gene is associated with an increased chance of a positive outcome of hepatitis B treatment.
In yet another embodiment, a method is provided wherein it is also determined whether said individual has a serum HBsAg baseline content of less than 387 IU/mL before start of hepatitis B treatment. A serum HBsAg baseline content of less than 387 IU/mL could be used as another parameter that is predictive for a positive outcome of hepatitis B treatment (R.B. Takkenberg, thesis University of Amsterdam (2011), The Netherlands, chapter 2, ISBN 9789090263045). Hence, a combination of a method according to the invention and determination of the HBsAg baseline content has an even increased predictive value for hepatitis B treatment outcome.

The terms "individual," "patient", "host" and "subject" are used interchangeably herein to refer to a vertebrate, preferably a mammal, more preferably a human.

The present invention employs, unless otherwise indicated, conventional techniques of molecular biology (including recombinant techniques), microbiology, cell biology, biochemistry and immunology, which are within the skill of the art.

As used herein, the singular form of any term can alternatively encompass the plural form and vice versa.

As used herein, a carnitine derivative preferably comprises acetyl-L-carnitine and/or proprionyl-L-carnitine.

The invention is further illustrated by the following examples. These examples are not limiting the invention in any way, but merely serve to clarify the invention.

### Brief description of the drawings

Figure 1: HBV treatment outcome-associated SNPs. Each corresponding sequence can be found in dbSNP by their RefSNP accession ID (rs number). For example http://www.ncbi.nlm.nih.gov/snp?term=rs12356193. The allele of each SNP that is associated with corresponding HBV treatment outcome is shown in a separate column (column 6).
Figure 2: positive HBV treatment outcome-associated HBV alleles. The allele of each SNP that is associated with corresponding HBV treatment outcome is shown in a separate column (column 4) of Figure 2a.
Figure 3: Plot of GWA analysis for HBsAg loss at week 96 (y-axis: -log 10 of p-value using the Cochran Armitage test for trend, x-axis: position on genome). rs12356193 is depicted in the circle.
Figure 4: Q-Q plot of HBsAg loss at week 96.
Figure 5: Percentage of HBsAg loss in different rs12356193 genotypes.
Figure 6: rs12356193 allelic distribution in patients with and without HBsAg loss.
Figure 7:
   a). Distribution of rs12356193 genotypes among patients with viral genotype A, D and E.
   b). Percentage of HBsAg loss in different rs12356193 genotypes in patients with viral genotype A, D and E.

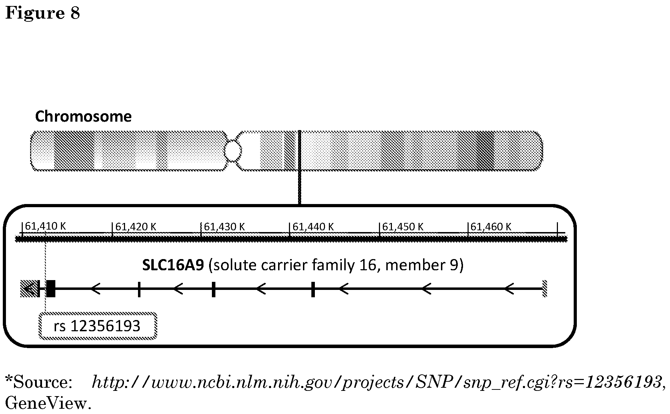

Figure 8: Schematic representation of location of SNP rs12356193 on chromosome 10.

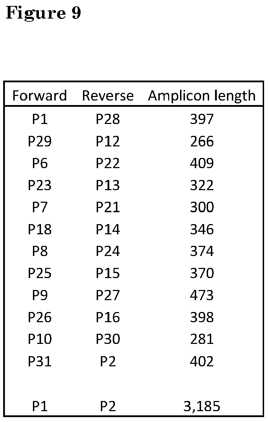

Figure 9: Sequences of PCR fusion primers.

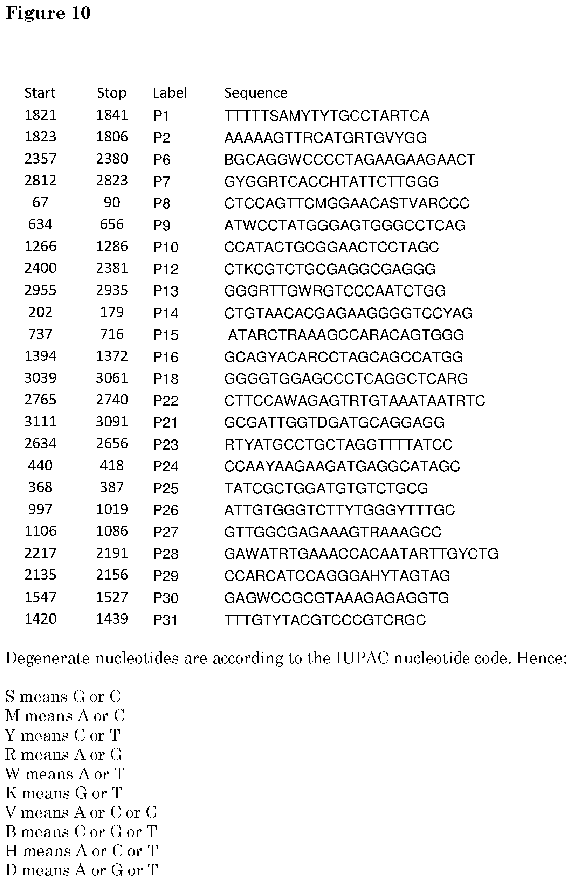

Figure 10: Combinations of primers and resulting amplicon length.

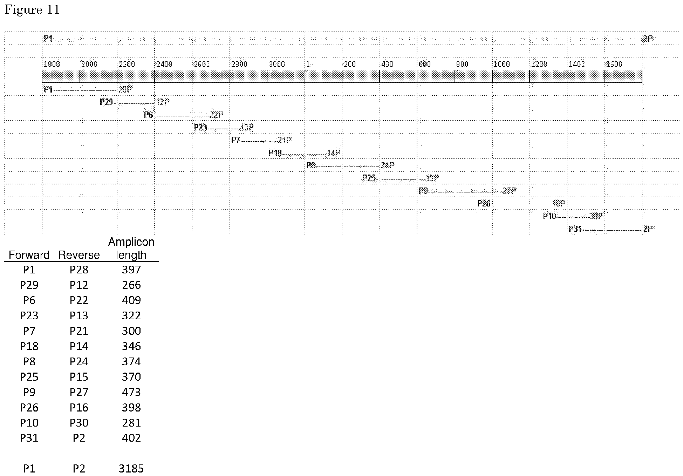

Figure 11: Schematic sequencing workflow.
Figure 12: Mean levels of DL-Carnitine (C0), Acetyl-L-Carnitine (C2) and Propionyl-L-Carnitine (C3) for different rs12356193 genotypes.
Figure 13: Mean levels of DL-Carnitine (C0), Acetyl-L-Carnitine (C2) and Propionyl-L-Carnitine (C3) for patients with HBsAg loss at 2 years of treatment free follow up and patients with HBsAg persistence at 2 years of treatment free follow up.

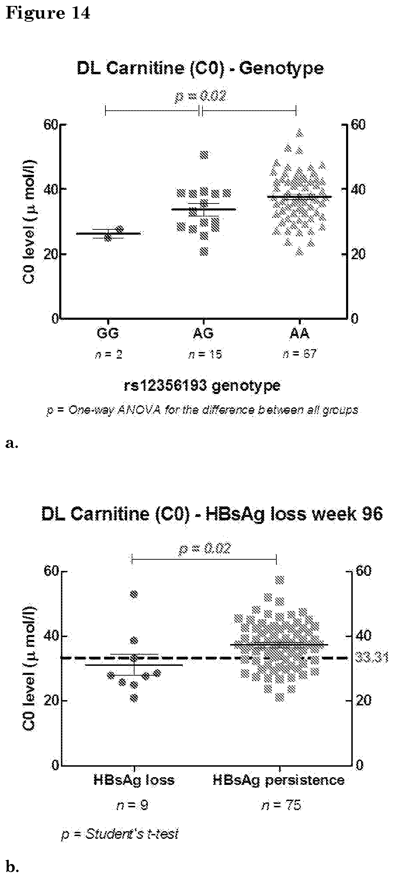

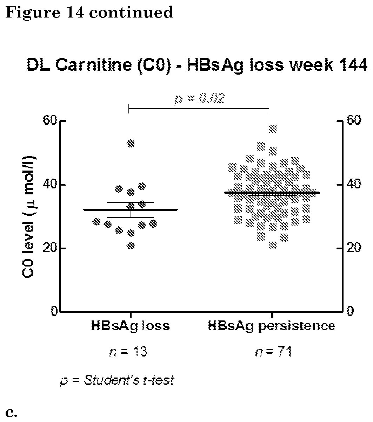

Figure 14: Plasma DL-Carnitine (C0) levels according to rs12356193 genotype in all 84 patients (a); Plasma DL-Carnitine (C0) levels in patients with and without HBsAg loss at week 96 (b) and at week 144 (c).

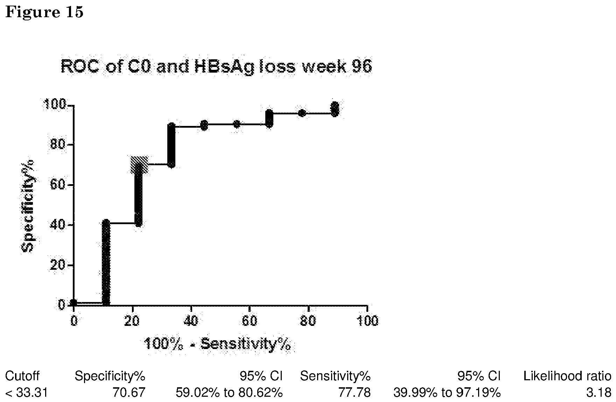

Figure 15: ROC curve of baseline DL-Carnitine (C0) levels in relation with HBsAg loss at week 96 in all patients.

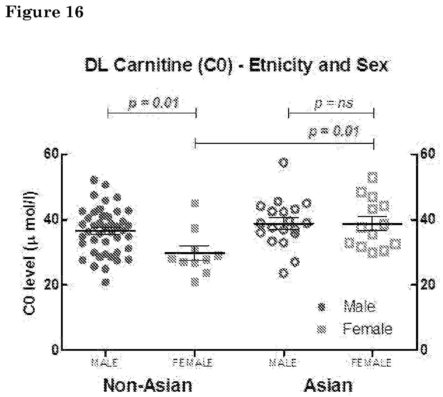

Figure 16: Plasma DL-Carnitine (C0) level in patients according to ethnicity and sex.

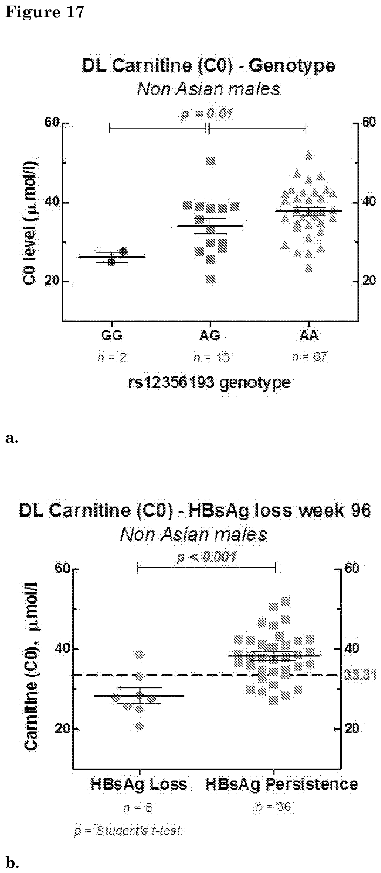

Figure 17: a) Plasma DL-Carnitine (C0) levels according to rs12356193 genotype in non-Asian male patients; b) Plasma DL-Carnitine (C0) levels in non-Asian male patients with and without HBsAg loss at week 96.

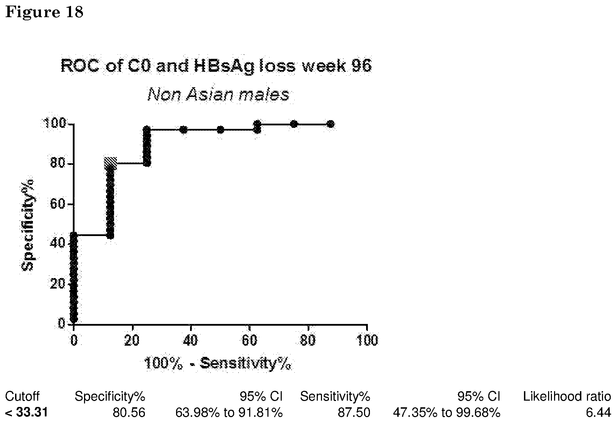

Figure 18: ROC curve of baseline DL-Carnitine (C0) levels in relation with HBsAg loss at week 96 in non-Asian male patients.

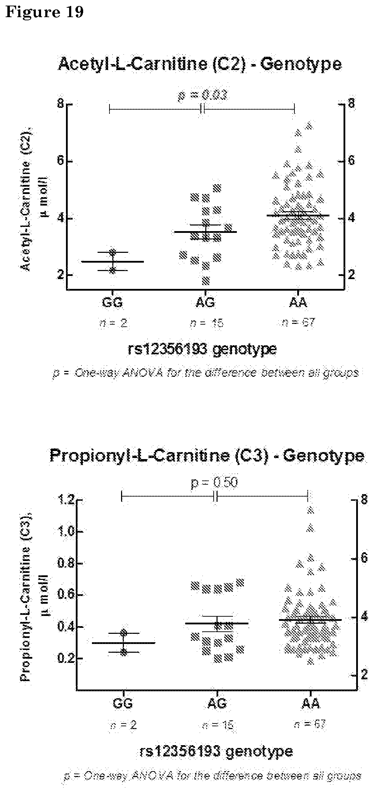

Figure 19: Plasma Acetyl-L-Carnitine (C2) and Plasma Propionyl-L-Carnitine (C3) levels according to rs12356193 genotype in all 84 patients.

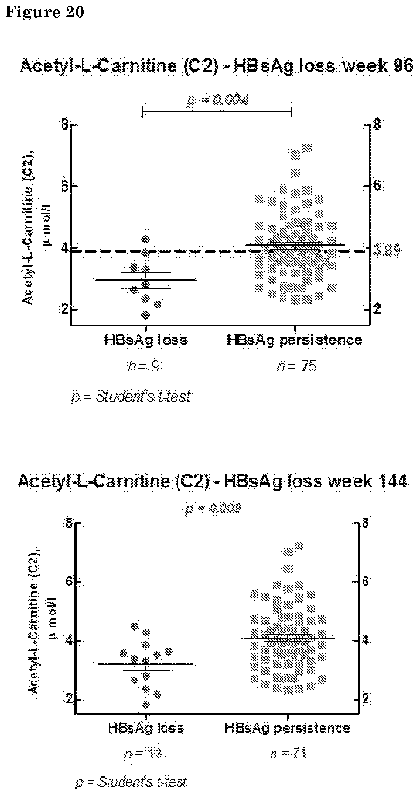

Figure 20: Plasma Acetyl-L-Carnitine (C2) levels in patients with and without HBsAg loss at week 96 and at week 144.

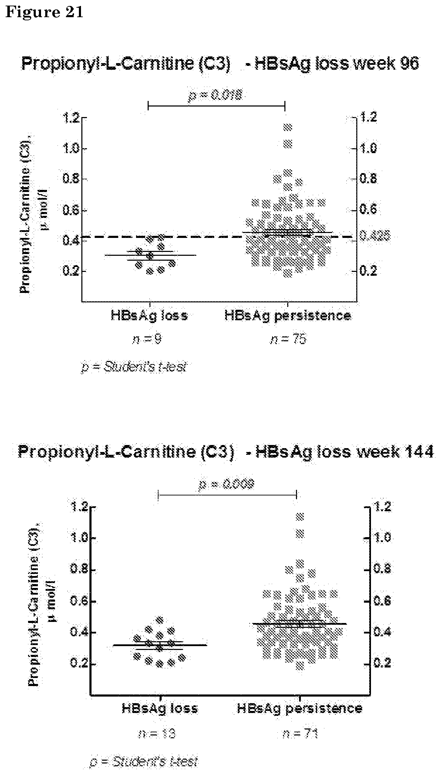

Figure 21: Plasma Propionyl-L-Carnitine (C3) levels in patients with and without HBsAg loss at week 96 and at week 144.

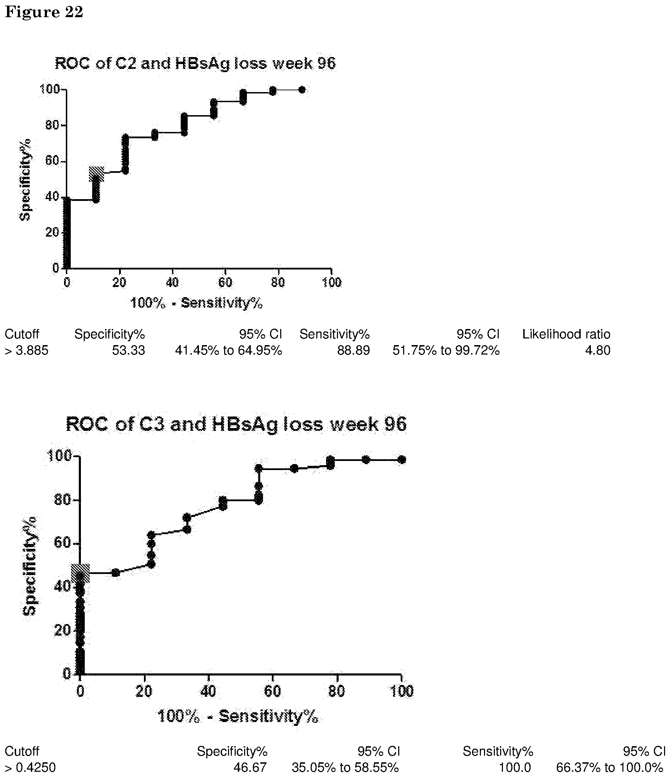

Figure 22: ROC curve of baseline Acetyl-L-Carnitine (C2) and Propionyl-L-Carnitine (C3) levels in relation with HBsAg loss at week 96 in all patients.

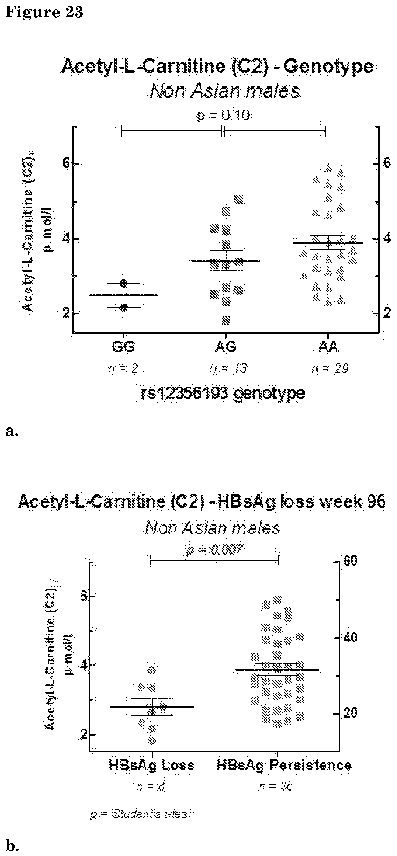

Figure 23: (a) Plasma Acetyl-L-Carnitine (C2) levels according to rs12356193 genotype in non-Asian male patients; (b) Plasma Acetyl-L-Carnitine (C2) levels in non-Asian male patients with and without HBsAg loss at week 96.

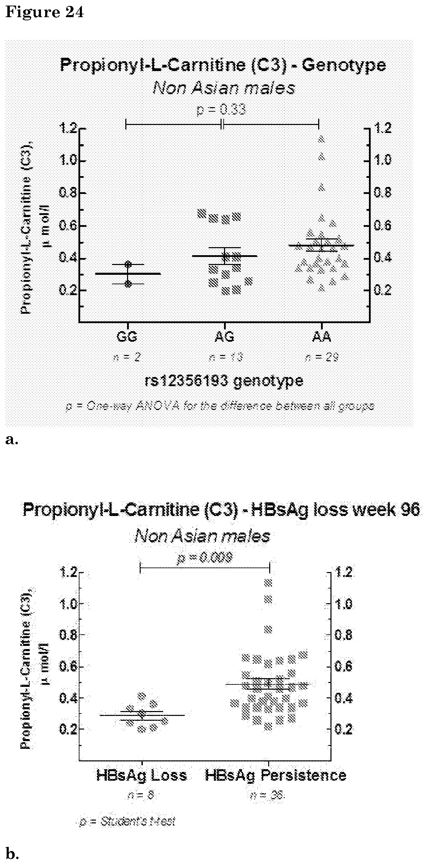

Figure 24: (a) Plasma Propionyl-L-Carnitine (C3) levels according to rs12356193 genotype in non-Asian male patients; (b) Plasma Propionyl-L-Carnitine (C3) levels in non-Asian male patients with and without HBsAg loss at week 96.

### Examples

### Example 1

We analyzed genome wide association data of 84 chronic active hepatitis B patients treated with peginterferon and adefovir to identify associations between single nucleotide polymorphisms (SNP) and treatment outcome.

### Patients-Methods

### 1. PEG/ADV study method

In a clinical study recently performed at the AMC [(R.B. Takkenberg, thesis University of Amsterdam (2011), The Netherlands, chapter 2, ISBN 9789090263045)]. 84 chronic hepatitis B patients (40 HBeAg positive; 44 HBeAg negative) with HBV DNA ≥ 2x10⁴ IU/mL were treated with Peg INF alfa-2a (Pegasys®) and ADV-dipivoxil (Hepsera®) for 48 weeks, followed by a 24-week (and up to 5 years) treatment-free follow-up.

Response definitions were HBeAg seroconversion (loss of HBeAg with appearance of anti-HBe antibodies), SVR (HBV DNA levels ≤ 2,000 IU/mL and normalization of ALT), and HBsAg seroconversion (loss of HBsAg with the appearance of anti-HBs antibodies) during follow-up.

screening Peg interferon + Adefovir Follow-up
0wk 48wk 72wk 5yr

### 2. Baseline characteristics patients

There was a marked heterogeneity of ancestry across the study population. Reflected in a genotype distribution of A (26), B (14), C (12), D (23) and E (9). Sustained virological response (SVR), was achieved in 32 patients. HBeAg seroconversion occurred during treatment in 14 HBeAg positive patients. Loss of serum hepatitis B surface antigen (HBsAg), defined as HBsAg levels under the lower detection limit of quantification (0.05 IU/mL) using the Abbot Architect, was achieved in 9 patients at end of follow up (11%).

No statistically significant differences in baseline characteristics were found when comparing all patients. However, in HBeAg positives older age was positively associated with HBsAg seroconversion (OR 1.16 per 1 year increase at baseline [95%CI, 1.008-1.337] p=0.039). In HBeAg negatives both low baseline HBsAg (OR 16.96 per 1log10 IU/mL decline [95% CI, 2.85-101] p=0.002) and HBV DNA (OR 2.36 per 1log10 IU/mL decline [95% CI, 1.02-5.45] p=0.044) were associated with an increased probability of HBsAg seroconversion. In multivariate analysis, HBsAg was the only independent predictor of HBsAg seroconversion (OR 17.00 per 1log10 IU/mL decline [95% CI 2.27-127, p=0.006). [As described by (R.B. Takkenberg, thesis University of Amsterdam (2011), The Netherlands, chapter 2, ISBN 9789090263045)].

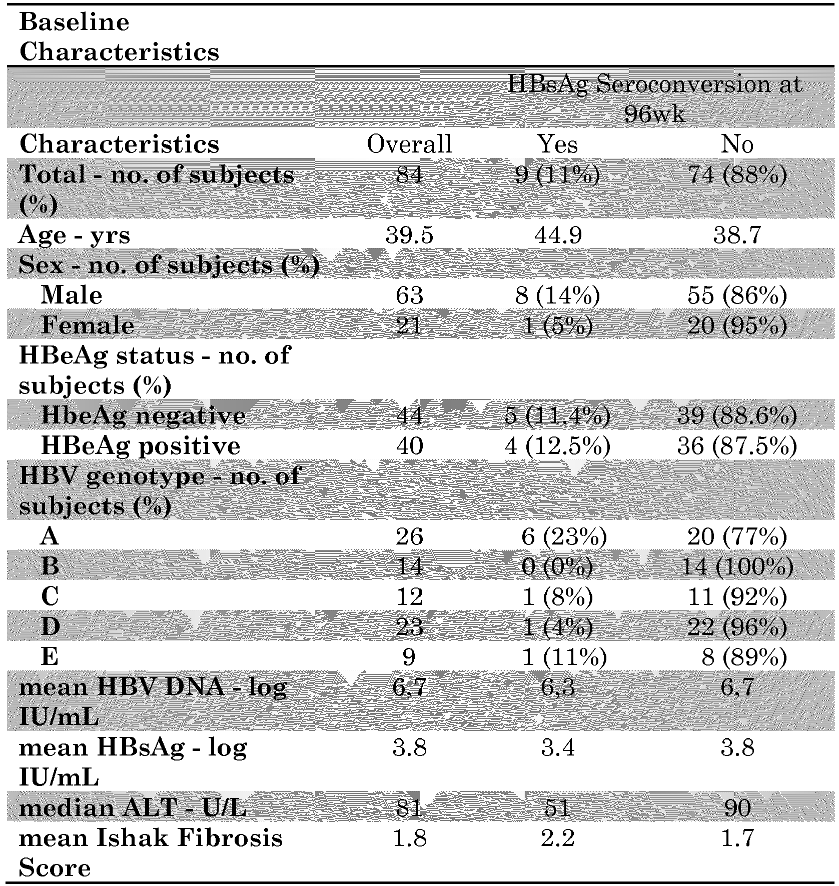

### 3. Genotyping

### DNA sample preparation (200 ng DNA input)

Genotyping of 84 individuals was performed at Roche (Nutley) using the Illumina Human Omni1-Quad BeadChip (Illumina, Inc. San Diego, USA).

Bead intensity data were processed and normalized in BeadStudio (Illumina); data for successfully genotyped samples were extracted and genotypes called within collections using Illuminus.

### 4. GWAS & Statistics

GWA analysis was performed using the GenABEL* package in R Statistical Software. * Aulchenko YS, Ripke S, Isaacs A, van Duijn CM. GenABEL: an R library for genome-wide association analysis. Bioinformatics (2007) 23 (10): 1294-1296.

In total 999,091 different SNPs were genotyped in 84 people. After quality control 204,862 (20.5%) SNPs were excluded as having low (<5%) minor allele frequency, 86 (0.009%) SNPs because of low call rate (>5% missing data) and 31 (0.003%) SNPs because they were out of Hardy-Weinberg equilibrium (P <1e-10). No patients were excluded because of low (<95%) call rate, too high autosomal heterozygosity (FDR <1%) or too high IBS (>=0.95). In total 794,130 (79.5%) SNPs and 84 (100%) people passed all criteria, and were used for further analysis.

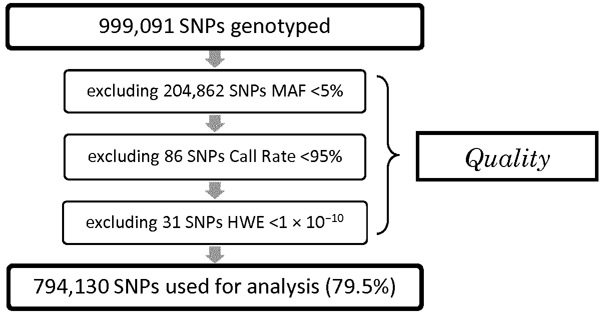

Association analysis was performed on loss of HBsAg one year of treatment free follow up (week 96) in the total study population. In addition, association analyses were performed on other treatment outcomes (HBeAg seroconversion, SVR and HBsAg decline at week 24) in all patients combined and in patients clustered for ancestry or viral genotype separately.

There was some inflation of summary statistics, which could be indicative of population stratification (see Q-Q plot HBsAg loss week 96). Lambda X values ranged from 0.97 to 1.07, implying a possible effect on positive associations due to population stratification.

Statistical significance of the association with each SNP was assessed using a 1-degree-of-freedom Cochrane-Armitage trend test. We applied a conservative Bonferroni correction to control for false-positive error rates deriving from multiple testing, and therefore considered genome wide associations with a P value of < 6.3 x 10E-8 (=0,05 / 794,161) to be genome wide significant. Odds ratios and confidence intervals were calculated using the major allele as a reference. HWE p-values were calculated in GenABEL.

### Results

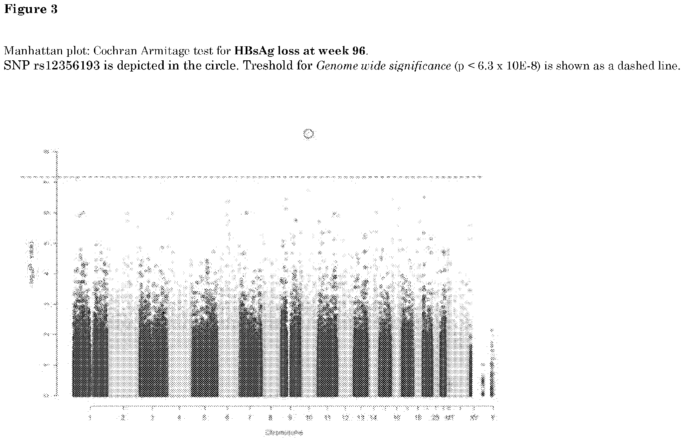

Figure 3 shows a -log₁₀ *P* value plot. P values were calculated by 1-d.f. Cochrane-Armitage trend test. The large circled dot on the chromosome 10 showed a genome wide significant association of SNP rs12356193 with HBsAg loss at one year of treatment free follow up (week 96).

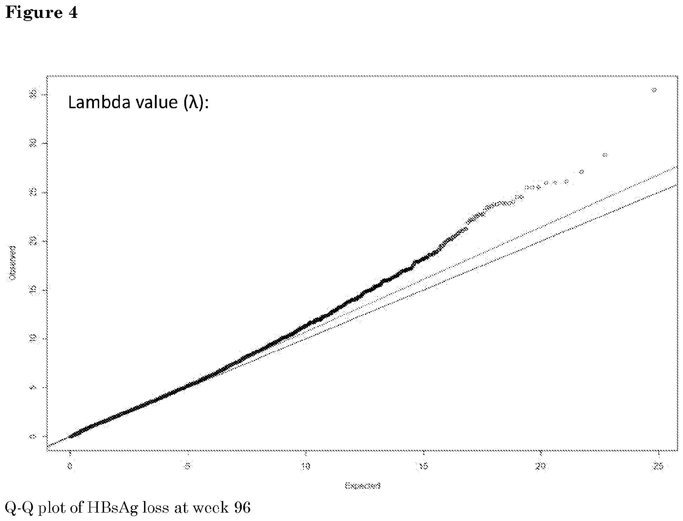

Figure 4 shows a Q-Q plot of HBsAg loss at week 96

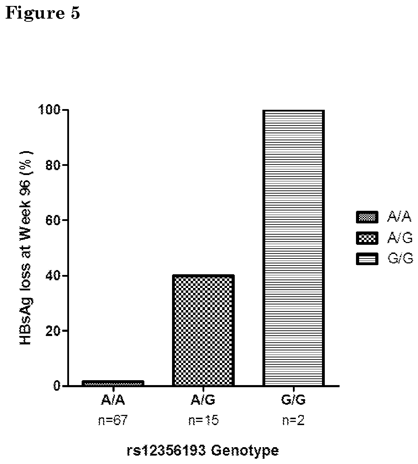

Figure 5 shows the percentage of HBsAg loss in different rs12356193 genotypes.

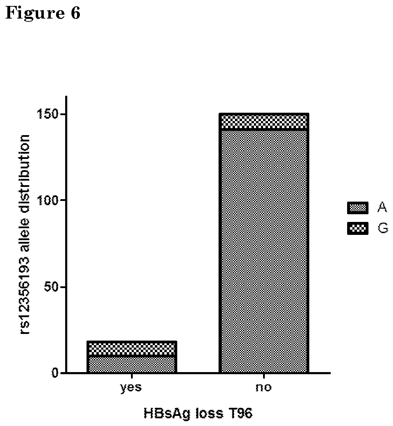

Figure 6 shows rs12356193 allelic distribution in patients with and without HBsAg loss.

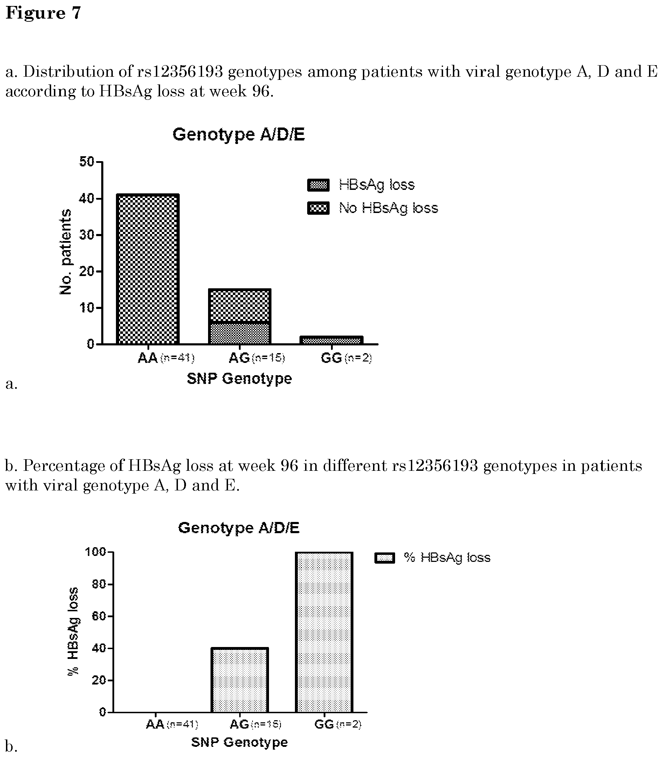

Figure 7 shows the distribution of rs12356193 genotypes among patients with viral genotype A, D and E and the percentage of HBsAg loss in different rs12356193 genotypes in patients with viral genotype A, D and E.

Figure 8 shows a schematic representation of location of SNP rs12356193 on chromosome 10.

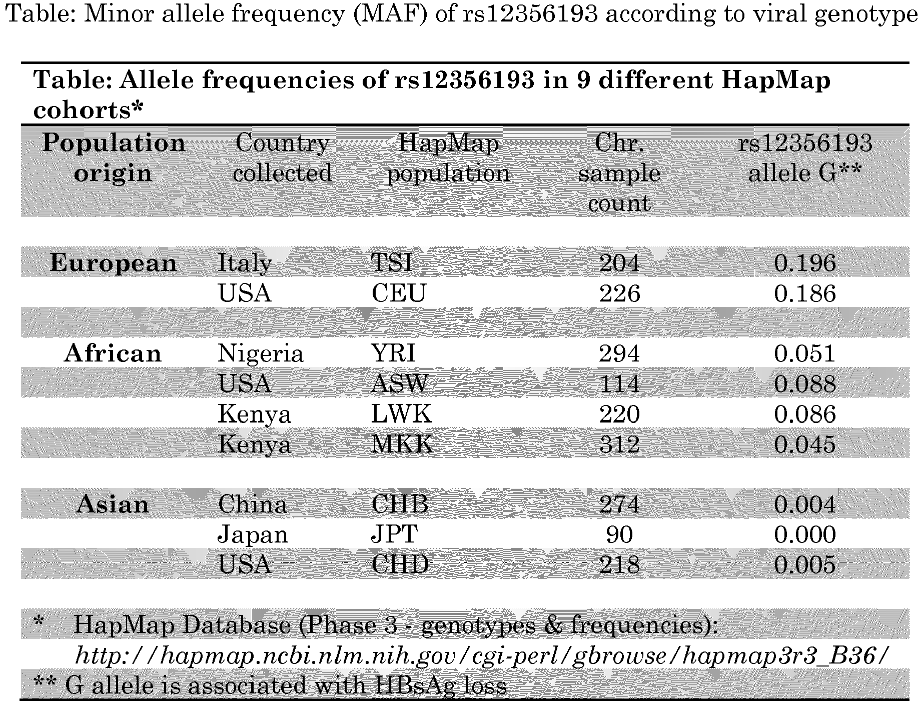

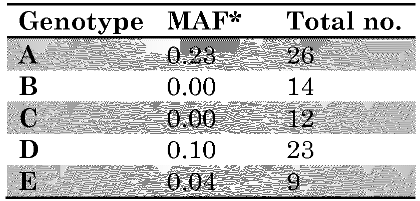

### Discussion

The minor allele of one SNP, rs12356193, was significantly associated with HBsAg loss at one year of follow up (figure), with a p-value of 2.61E-09 and an overall minor allele frequency of 0.12. This SNP is located on chromosome 10 in the SLC16A9 gene. Figure 1 also shows other SNPs that are associated with hepatitis B treatment outcome, which are suitable for increasing the predictive value. Alleles associated with positive outcomes are depicted in column six.

The clinical significance of these findings is two-fold;
1. First, risk stratification of patients through SNP genotyping helps guide therapeutic decision- making. Identifying those at greatest chance of positive outcome such as HBsAg loss, SVR or more than 1.5 log HBsAg decline at week 24 will assist decisions of whether or not to start with peginterferon based treatment.
2. Second, the novel association of the SLC16A9 loci with chronic hepatitis B suggests an important role for this gene in the response to HBV treatment with peginterferon and adefovir. This association provides new insight into the host immune response to hepatitis B infection and offers new potential targets for therapeutic agents.

### Example 2

We analyzed HBV sequence data of 84 chronic active hepatitis B patients treated with PEGinterferon and adefovir to identify associations between single nucleotide polymorphisms (SNP) and treatment outcome.

### Patients-Methods

### 1. PEG/ADF study method

In a clinical study recently performed at the AMC [B. Takkenberg *et al.* (R.B. Takkenberg, thesis University of Amsterdam (2011), The Netherlands, chapter 2, ISBN 9789090263045)] 84 chronic active hepatitis B patients (40 HBeAg positive; 44 HBeAg negative) were treated with Peg INF alfa-2a (Pegasys®) and ADF-dipivoxil (Hepsera®) for 48 weeks, followed by a 24-week (and up to 5 years) treatment-free follow-up.

### 2. Baseline characteristics patients

There was a marked heterogeneity of ancestry across the study population. Reflected in a genotype distribution of A (26), B (14), C (12), D (23) and E (9). Sustained virological response (SVR), was achieved in 32 patients. HBeAg seroconversion occurred during treatment in 14 HBeAg positive patients. Loss of serum hepatitis B surface antigen (HBsAg), defined as HBsAg levels under the lower detection limit of quantification (0.05 IU/mL) using the Abbot Architect, was achieved in 9 patients at end of follow up (11%).

### 3. Sample preparation

A set of PCR fusion primer sets were designed to amplify 13 amplicons and to cover the HBV genome. Sequence-specific primers were designed with degenerate bases in positions to accommodate variations in HBV reference sequences for genotypes A, B, C, D and E (Entrez identifiers X02763, D00330, AY123041, J02203, X75657 respectively). The resulting PCR fragments were immobilized onto DNA Capture Beads via a unique single-stranded region to enable the attachment of the fragments at a 1:1 ratio to beads; i.e. each fragment was attached to a single bead.

### 4. Emulsion PCR Amplification and sequencing

The bead-bound library was emulsified with amplification reagents in a water-in-oil mixture resulting in microreactors containing just one bead with one unique sample-library fragment. Each unique sample-library fragment was amplified in parallel within its own microreactor, excluding competing or contaminating sequences. The DNA-carrying capture beads were loaded onto PicoTiterPlate device for sequencing each bead in a well.
After loading the PicoTiterPlate device into a 454 Genome Sequencer FLX Instrument, individual nucleotides were flowed in a fixed order across the open wells and DNA Capture Beads. Addition of one (or more) nucleotide(s) complementary to the template strand resulted in a chemiluminescent signal recorded by the CCD camera of the Genome Sequencer FLX Instrument. The entire workflow of PCR and sequencing was performed by 454 Life Sciences, Branford CT.

Figures 9, 10 and 11 show the primers, amplicons and a schematic outline of sequencing protocol.

### 5. Alignment and Statistics

Patient specific HBV sequences were aligned using ClustalW Multiple Alignment in BioEdit sequence analysis software*.
* Hall, T.A. 1999. BioEdit: a user-friendly biological sequence alignment editor and analysis program for Windows 95/98/NT. Nucl. Acids. Symp. Ser. 41:95-98.

Nucleotides at each position in each patient were compared against a genotype specific reference sequence. Sequences used as reference were A adw2 (X02763), Ba ad (D00330), C (AB033556), D ayw (X02496) and E (X75657) for genotypes A, B, C, D and E respectively. Positions where at least three or more mutations were present in all patients were used for further analysis. In total 503 positions were analyzed using frequency cross tables and Chi square tests for proportions.

We applied a conservative Bonferroni correction to control for false-positive error rates deriving from multiple testing, and therefore considered associations with a *P* value of < 9.9 x 10E-5 (=0,05 / 503) to be statistically significant.

Association analysis was performed on loss of HBsAg at one year of treatment free follow up (week 96) in the total study population. In addition, association analyses were performed on other treatment outcomes (HBeAg seroconversion, SVR and HBsAg decline at week 24) in all patients combined and in patients clustered for ancestry or viral genotype separately.

### Results

One HBV mutation was found to be significantly associated with HBsAg loss at week 96 after Bonferroni correction for multiple testing (Figure 2). This mutation occurred in 4 out of 9 patients who achieved HBsAg loss at week 96 and in none of the patients who did not lose HBsAg. Figure 2 also shows other HBV mutations that are associated with hepatitis B treatment outcome, which are suitable for increasing the predictive value. For instance, analyzing the presence of either the T784G or the G1888W mutation in combination dramatically improved the association (Figure 2).

### Discussion

Nucleotide position 784 is located in the S and Polymerase gene Open Reading Frame (ORF). In the S ORF a mutation at this position causes an amino acid substitution of Serine (S) into Arginine (R) (Figure 2). This mutation in the viral genome is, therefore, a marker for therapy response.

### Example 3

In Example 1 we identified a strong association between the minor allele (G) of a single nucleotide polymorphism (SNP) and HBsAg loss at one year of treatment free follow up in these patients. This SNP, rs12356193, is located on chromosome 10 in an intronic region of the SLC16A9 gene. Interestingly, presence of this SNP has been described to be strongly associated with L-carnitine levels in other GWA studies (Kolz et al., 2009). However, no studies have reported an effect of L-carnitine levels on HBsAg loss in chronic hepatitis B patients treated with peginterferon and/or adefovir. Therefore we aimed to determine L-carnitine levels in CHB patients treated with peginterferon and adefovir, and look for associations with rs12356193 genotype and HBsAg loss.

### Patients

A subset of 21 patients was selected for measurement of DL-Carnitine (C0), Acetyl-L-Carnitine (C2) and Propionyl-L-Carnitine (C3) (see methods section).

Baseline characteristics of patients included in this subset is shown in Table 1. In total, 2 patients had genotype GG for SNP rs12356193, 10 patients genotype AG and 9 patients genotype AA. No significant differences were present in baseline characteristics between patients with genotype AG or GG and patients with genotype AA.

**Table 1.**

| **Baseline characteristics** | **Genotype AG/GG** | | **Genotype AA** | | p |
|---|---|---|---|---|---|
| Number | 12 | | 9 | | |
| Mean age (years) (SD) | 41 | (7.3) | 38 | (6.7) | 0.26 |
| Female sex (%) | 0 | | 0 | | |
| Ancestry | | | | | |
| Caucasian (%) | 8 | (67) | 4 | (44) | 0.40 |
| African (%) | 4 | (33) | 5 | (56) | 0.40 |
| Median ALT (xULN) (iqr) | 1.7 | (0.8-5.2) | 2.9 | (1.9-5.0) | 0.58 |

| **Viral characteristics** | | | | | |
|---|---|---|---|---|---|
| HBeAg positive (%) | 6 | (50) | 4 | (44) | 1.00 |
| Mean HBV DNA (log10 IU/ml) (SD) | 6.47 | (2.0) | 6.94 | (2.0) | 0.60 |
| Mean HBsAg (log10 IU/ml) (SD) | 3.52 | (1.2) | 4.14 | (0.7) | 0.18 |
| HBV genotype | | | | | |
| A (%) | 8 | (67) | 6 | (67) | 1.00 |
| D (%) | 3 | (25) | 2 | (22) | 1.00 |
| E (%) | 1 | (8) | 1 | (11) | 1.00 |

| **Treatment outcome** | | | | | |
|---|---|---|---|---|---|
| HBsAg loss | 8 | (67) | 0 | | **0.005** |

### Methods

L-carnitine and carnitine derivate levels from baseline plasma samples were measured at the department of Genetic Metabolic Disorders (GMZ) at the AMC by Tandem Mass Spectometry according to a standardized protocol.

HBsAg loss in this subset was defined as undetectable HBsAg levels by Abbott AxSYM (HBsAg < 0.05 IU/mL). L-carnitine and carnitine derivate levels are shown in micromol per ml and differences of means were tested using an unpaired t-test. P values below 0.05 were considered statistically significant.

### Results

Mean levels of DL-Carnitine (C0), Acetyl-L-Carnitine (C2) and Propionyl-L-Carnitine (C3) for different rs12356193 genotypes are depicted in figure 12 and Table 2. Patients with rs12356193 genotype AG or GG had significantly lower levels of C0, C2 and C3 than patients with genotype AA.

Mean levels of DL-Carnitine (C0), Acetyl-L-Carnitine (C2) and Propionyl-L-Carnitine (C3) for patients with HBsAg loss at 2 years of treatment free follow up and patients with HBsAg persistence at 2 years of treatment free follow up are depicted in figure 13 and Table 2. Patients with HBsAg loss had significantly lower levels of C0, C2 and C3 than patients with HBsAg persistence.

**Table 2.**

| | **Genotype AG/GG** | | **Genotype AA** | | p |
|---|---|---|---|---|---|
| Number | 12 | | 9 | | |
| Mean DL-Carnitine; C0 (µmol/ml) (SD) | 30.4 | (1.7) | 40.1 | (1.0) | <0.001 |
| Mean Acetyl-L-Carnitine; C2 (µmol/ml) (SD) | 2.9 | (0.2) | 4.0 | (0.3) | 0.004 |
| Mean Propionyl-L-Carnitine; C3 (µmol/ml) (SD) | 0.33 | (0.04) | 0.50 | (0.03) | 0.003 |

| | **HBsAg loss** | | **HBsAg persistence** | | |
|---|---|---|---|---|---|
| Number | 8 | | 13 | | |
| Mean DL-Carnitine; C0 (µmol/ml) (SD) | 28.4 | (1.9) | 38.4 | (1.3) | <0.001 |
| Mean Acetyl-L-Carnitine; C2 (µmol/ml) (SD) | 2.8 | (0.2) | 3.8 | (0.2) | 0.014 |
| Mean Propionyl-L-Carnitine; C3 (µmol/ml) (SD) | 0.29 | (0.03) | 0.47 | (0.03) | <0.001 |

### Conclusion

In a GWAS of 84 CHB patients treated with peginterferon and adefovir, we identified a strong association between the minor allele (G) of a SNP (rs12356193) and HBsAg loss. We confirmed that levels of DL-Carnitine (C0), Acetyl-L-Carnitine (C2) and Propionyl-L-Carnitine (C3) were significantly associated with different rs12356193 genotypes. In addition we showed that patients with HBsAg loss had significantly lower levels of C0, C2 and C3 than patients with HBsAg persistence.

### Example 4

In Example 3, we confirmed that levels of DL-Carnitine (C0), Acetyl-L-Carnitine (C2) and Propionyl-L-Carnitine (C3) were significantly associated with different rs12356193 genotypes and that patients with HBsAg loss had significantly lower levels of C0, C2 and C3 than patients with HBsAg persistence.

In this Example, we extended the study population to 84 patients and in addition assessed plasma DL-Carnitine (C0), Acetyl-L-Carnitine (C2) and Propionyl-L-Carnitine (C3) levels as predictor of HBsAg loss and association of C0, C2 and C3 levels with HBsAg loss in a subpopulation of non-Asian males.

### Methods (Carnitine measurement by MS-MS)

To assess concentrations of plasma carnitine and carnitine derivates, a Quattro II triple-quadrupole mass spectrometer (Micromass, Manchester, UK) in the negative electrospray ionization (ESI) mode and the micromass MassLynx data system was used with 50 micro liters input at the laboratory of Genetic Metabolic Disorders (AMC, Amsterdam, the Netherlands), following the method as described earlier (Chase et al. 1997).

### Patients

Baseline characteristics of patient are indicated in Table 3.

**Table 3. Baseline characteristics of all HBeAg-positive and -negative patients included in GWAS analysis.**

| **Characteristics** | **Main study population (*n* = 84)** | | **HBsAg loss (*n* = 9)** | | **HBsAg persistence** | | p* |
|---|---|---|---|---|---|---|---|
| ***Demographics*** | | | | | | | |
| Mean age, years (SD) | 39.5 | (10.3) | 44.9 | (11.8) | 38.9 | (10.0) | 0.10 ¹ |
| Female, *n* (%) | 21 | (25) | 1 | | 20 | | 0.44 ³ |
| Etnicity | | | | | | | 0.18 ³ |
| Caucasian, *n* (%) | 27 | (32) | 5 | (56) | 22 | (29) | |
| African, *n* (%) | 26 | (31) | 3 | (33) | 23 | (31) | |
| Asian, *n* (%) | 31 | (37) | 1 | (11) | 30 | (40) | |

| ***Laboratory characteristics*** | | | | | | | |
|---|---|---|---|---|---|---|---|
| Median ALT, xULN (iqr) | 1.8 | (1.1-3.2) | 1.5 | (0.8-4.0) | 2.0 | (1.1-3.2) | 0.31 ² |
| HBeAg positive, *n* (%) | 40 | (48) | 4 | | 36 | | 1.00 ³ |
| Mean HBV-DNA, log₁₀ IU/ml (SD) | 6.67 | (1.73) | 6.18 | (2.22) | 6.73 | (1.67) | 0.37 ¹ |
| Mean HBsAg, log₁₀ IU/ml (SD) | 3.78 | (0.87) | 3.30 | (1.30) | 3.84 | (0.79) | 0.25 ¹ |
| HBV genotype | | | | | | | 0.13 ³ |
| A, *n* (%) | 26 | (31) | 6 | (67) | 20 | (27) | |
| B, *n* (%) | 14 | (17) | 0 | (0) | 14 | (19) | |
| *C, n* (%) | 12 | (14) | 1 | (11) | 11 | (15) | |
| *D, n* (%) | 23 | (27) | 1 | (11) | 22 | (29) | |
| E, *n* (%) | 9 | (11) | 1 | (11) | 8 | (11) | |

| ***Response at week 96*** | | | | | | | |
|---|---|---|---|---|---|---|---|
| HBsAg loss, *n* (%) | 9 | (11) | - | | - | | |
| Combined response, *n* (%) | 25 | (30) | - | | - | | |

| ***Baseline Carnitine level*** | | | | | | | |
|---|---|---|---|---|---|---|---|
| DL-Carnitine (C0), µ mol/ml (SD) | - | | 31.1 | (9.6) | 37.3 | (7.1) | **0.02** ¹ |
| Acetyl-L-Carnitine (C2), µ mol/ml (SD) | - | | 2.95 | (0.81) | 4.08 | (1.09) | **0.004** ¹ |
| Propionyl-L-Carnitine (C3), µ mol/ml | - | | 0.30 | (0.08) | 0.45 | (0.18) | **0.02** ¹ |

### Results

### 1. Association of plasma DL-Carnitine (C0) levels with SNP rs12356193 and HBsAg loss

SNP rs12356193 is located on chromosome 10 in an intronic region of the SLC16A9 gene. This SNP is reported to be strongly associated with plasma DL-Carnitine (C0) levels in other GWAS studies (Kolz et al. 2009) (β -3.58, p = 4.0x10⁻²⁶). This association was confirmed in our complete study cohort (n = 84), as mean plasma DL-Carnitine (C0) levels were significantly different in patients with the GG, AG and AA genotype (26.3 vs 33.7 vs 37.7 µmol/L respectively, p = 0.02, figure 14a and table 3). Plasma DL-Carnitine (C0) levels were also lower in patients with HBsAg loss at week 96, than patients with HBsAg persistence (31.1 vs 37.3 µmol/L, p = 0.02, figure 14b). Treatment outcome data on all patients treated in our study up to 2 years after end of treatment (week 144) are available. Plasma DL-Carnitine (C0) levels were also lower in patients with HBsAg loss at week 144, than patients with HBsAg persistence (32.2 vs 37.4 µmol/L, p = 0.02, figure 14c).

### 2. Plasma DL-Carnitine (C0) levels as predictor of HBsAg loss at week 96

Discrimination of variables defined as the ability to distinguish patients who will achieve HBsAg loss at week 96 from those who will not, was assessed by area under the receiver operator characteristic curve (AUC) analysis.

Figure 15 shows the AUC for the prediction of HBsAg loss at week 96, which was 0.75 (95%CI, 0.54-0.96; p=0.01). Using baseline DL-Carnitine (C0) levels of ≤ 33.31 µmol/L as cut-off, the positive predictive value (PPV) was 24% and the negative predictive value (NPV) was 96% with a sensitivity of 78% and a specificity of 71%.

### 3. Sub-analysis of plasma DL-Carnitine (C0) levels in non-Asian males

Various factors are known to influence the level of plasma DL-Carnitine (C0), e.g. dietary intake, sex, age, and disease (Cederblad et al. 1976; Cederblad et al 1987; Flanagan 2010). In our cohort, DL-Carnitine (C0) level varied significantly in patients of different sex and ethnicity (figure 16).

Since ethnicity and sex appeared to be confounding factors for DL-Carnitine (C0) levels in our study, in addition to the finding of very low prevalence of the favorable SNP in Asians, we decided to further assess the association of DL-Carnitine (C0) levels and HBsAg loss in a subpopulation of non-Asian males (n=44).

### 4. Association of plasma DL-Carnitine (C0) levels with SNP rs12356193 and HBsAg loss in non-Asian males

Overall, the mean baseline DL-Carnitine (C0) level in non-Asian male patients was 36.5 µmol/L (SD 7.0). In non-Asian males, mean plasma DL-Carnitine (C0) levels were significantly different in patients with the GG, AG and AA genotype (26.3 vs 33.7 vs 38.5 µmol/L, respectively, p = 0.01, figure 17a).

In addition, plasma DL-Carnitine (C0) levels were lower in non-Asian males with HBsAg loss at week 96 than those with HBsAg persistence (28.4 vs 38.3 µmol/L, respectively, p < 0.001, figure 17b). Of note, all non-Asian patients with HBsAg loss were male (n=8) which made a sub-analysis of the association of DL-Carnitine (C0) levels and HBsAg loss in non-Asian females impossible.

### 5. Plasma DL-Carnitine (C0) levels as predictor of HBsAg loss at week 96 in non-Asian males

Figure 18 shows the AUC for the prediction of HBsAg loss, which was 0.75 (95%CI, 0.54-0.96; p=0.01). Using baseline DL-Carnitine (C0) levels of ≤ 33.31 µmol/L as a cut-off, the positive predictive value (PPV) was 50% and the negative predictive value (NPV) was 97% with a sensitivity of 88% and a specificity of 81%.

### 6. Association of plasma Acetyl-L-Carnitine (C2) and Propionyl-L-Carnitine (C3) levels with SNP rs12356193 and HBsAg loss

Acetyl-L-Carnitine (C2) and Propionyl-L-Carnitine (C3) are two esters of DL-Carnitine (C0) and also measured by the same Tandem Mass Spectometry method. Similar associations with SNP rs12345193 and HBsAg loss as with DL-Carnitine (C0) were found for these two esters.

Mean plasma Acetyl-L-Carnitine levels were significantly different in patients with the GG, AG and AA genotype (2.49 vs 3.51 vs 4.10 µmol/L respectively, p = 0.03, figure 19a), however, this association was not significant for Propionyl-L-Carnitine level (0.30 vs 0.42 vs 0.44 µmol/L respectively, p = 0.50, figure 19b).

Plasma Acetyl-L-Carnitine levels were also lower in patients with HBsAg loss at week 96, than patients with HBsAg persistence (2.95 vs 4.08 µmol/L, p = 0.004, figure 20a and table 3). The same association was found for plasma Propionyl-L-Carnitine level (0.30 vs 0.45 µmol/L, p = 0.02, figure 21a and table 3). Patients with HBsAg loss at week 144 also had significantly lower levels of both Acetyl-L-Carnitine (C2) (3.22 vs 4.09 µmol/L, p = 0.009, figure 20b), and Propionyl-L-Carnitine (C3) (0.32 vs 0.46 µmol/L, p = 0.009, figure 21b).

### 7. Plasma Acetyl-L-Carnitine (C2) and Propionyl-L-Carnitine (C3) level as predictor of HBsAg loss at week 96

Figure 22a shows the AUC for the prediction of HBsAg loss at week 96 using Acetyl-L-Carnitine (C2), which was 0.80 (95%CI, 0.65-0.94; p=0.003). Using baseline Acetyl-L-Carnitine (C2) levels of ≤ 3.89 µmol/L as cut-off, the positive predictive value (PPV) was 19% and the negative predictive value (NPV) was 98% with a sensitivity of 89% and a specificity of 53%.

Figure 22b shows the AUC for the prediction of HBsAg loss at week 96 Propionyl-L-Carnitine (C3), which was 0.78 (95%CI, 0.64-0.92; p=0.006). Using baseline Propionyl-L-Carnitine (C3) levels of ≤ 0.43 µmol/L as cut-off, the positive predictive value (PPV) was 18% and the negative predictive value (NPV) was 100% with a sensitivity of 100% and a specificity of 47%.

### 8. Sub-analysis of plasma Acetyl-L-Carnitine (C2) and Propionyl-L-Carnitine (C3) levels in non-Asian males

Sub-analysis of non-Asian males did not change the significance of our findings in Acetyl-L-Carnitine (C2) and Propionyl-L-Carnitine (C3), as shown in figures 23 and 24.

### References

Altschul 1990, J Mol Biol 215, 403
Aulchenko YS, Ripke S, Isaacs A, van Duijn CM. GenABEL: an R library for genome-wide association analysis. Bioinformatics (2007) 23 (10): 1294-1296
Cederblad G. Plasma DL-Carnitine (C0) and body composition. Clin Chim Acta. 1976 Mar 1;67(2):207-12
Cederblad G. Effect of diet on plasma DL-Carnitine (C0) levels and urinary DL-Carnitine (C0) excretion in humans. Am J Clin Nutr. 1987 Apr;45(4):725-9
Chace DH, et al. Rapid diagnosis of MCAD deficiency: quantitative analysis of octanoylcarnitine and other acylcarnitines in newborn blood spots by tandem mass spectrometry. Clin Chem. 1997 Nov;43(11):2106-13
Dienstag J.L. N Engl J Med 2008, 359; 1486-1500
Dowdy and Wearden, Statistics for Research, John Wiley & Sons, New York 1983
Flanagan JL, et al. Role of DL-Carnitine (C0) in disease. Nutr Metab (Lond). 2010 Apr 16;7:30. doi: 10.1186/1743-7075-7-30
Galibert F, et al. Nature. 1979 Oct 25;281(5733):646-50.)
Hall, T.A. 1999. BioEdit: a user-friendly biological sequence alignment editor and analysis program for Windows 95/98/NT. Nucl. Acids. Symp. Ser. 41:95-98
Kolz M et al. Meta-analysis of 28,141 individuals identifies common variants within five new loci that influence uric acid concentrations. PLoS Genet. 2009 Jun;5(6):e1000504.
Needleman 1970, J Mol Biol 48; 443; Smith 1981, Adv Appl Math 2, 482 Takkenberg, R.B.. Thesis University of Amsterdam (2011) The Netherlands. Chapter 2, ISBN 9789090263045
Wiley, John & Sons, Current Protocols in Molecular Biology N.Y (1989), 6.3.1-6.3.6

## Claims

1. A method for determining whether a hepatitis B patient has an improved chance of a positive outcome of hepatitis B treatment, as compared to the mean hepatitis B patient population, comprising determining whether a nucleic acid-containing sample of said hepatitis B patient comprises the G allele of SNP rs12356193.

2. Method according to claim 1, wherein said positive outcome is selected from the group consisting of HBsAg loss, HBsAg loss with anti-HBs seroconversion, sustained virological response and a more than 1.5 log HBsAg decline in the blood of said patient at or before week 24 of therapy.

3. Method according to claim 1 or 2, comprising determining SNP rs12356193 in combination with at least one other SNP as depicted in Figure 1 and/or Figure 2.

4. Method according to any one of claims 1-3, further comprising determining whether the expression level of carnitine in a plasma sample of said hepatitis B patient is equal to or less than 33.3 micromole/liter, preferably equal to or less than 29 micromole/liter, which is indicative for a responder, or more than 33.3 micromole/liter, which is indicative for a non-responder.

5. Method according to any one of claims 1-4, further comprising determining whether the expression level of acetyl-L-carnitine in a plasma sample of said hepatitis B patient is equal to or less than 3.89 micromole/liter, which is indicative for a responder, or more than 3.89 micromole/liter, which is indicative for a non-responder.

6. Method according to any one of claims 1-5, further comprising determining whether the expression level of proprionyl-L-carnitine in a plasma sample of said hepatitis B patient is equal to or less than 0.43 micromole/liter, which is indicative for a responder, or more than 0.43 micromole/liter, which is indicative for a non-responder.

7. Interferon, optionally in combination with an inhibitor of the viral DNA polymerase, for use in a method for treating hepatitis B, **characterized in that** a hepatitis B patient is treated that has been tested positive for the G allele of SNP rs12356193.

8. Interferon for use in a method for treating hepatitis B according to claim 7, wherein a hepatitis B patient is treated that has been tested positive for the G allele of SNP rs12356193, in combination with at least one other positive outcome-associated SNP allele as depicted in Figure 1 and/or Figure 2.

9. A method for determining whether an individual has a genetic predisposition for an improved chance for a positive outcome of hepatitis B treatment, as compared to the mean hepatitis B patient population, comprising determining whether genomic nucleic acid of said individual comprises at least the G allele of SNP rs12356193.

10. Method according to any one of claims 1-6 or 9, further comprising determining whether said individual has a HBsAg baseline content of less than 387 IU/mL before start of hepatitis B treatment.

## Patentansprüche

1. Verfahren zur Bestimmung, ob ein Hepatitis-B-Patient eine bessere Aussicht auf einen positiven Ausgang einer Hepatitis-B-Behandlung hat, verglichen mit der mittleren Hepatitis-B-Patientenpopulation, umfassend Bestimmen, ob eine nukleinsäurehaltige Probe des Hepatitis-B-Patienten das G-Allel von SNP rs12356193 umfasst.

2. Verfahren nach Anspruch 1, wobei der positive Ausgang ausgewählt ist aus der Gruppe bestehend aus HBsAg-Verlust, HBsAg-Verlust mit Anti-HBs-Serokonversion, anhaltender virologischer Reaktion und mehr als 1,5 log HBsAg-Abnahme im Blut des Patienten an oder vor der 24. Therapiewoche.

3. Verfahren nach Anspruch 1 oder 2, umfassend Bestimmen von SNP rs12356193 in Kombination mit mindestens einem anderen SNP, wie in Figur 1 und/oder Figur 2 dargestellt.

4. Verfahren nach einem der Ansprüche 1-3, ferner umfassend Bestimmen, ob das Expressionsniveau von Carnitin in einer Plasmaprobe des Hepatitis-B-Patienten gleich oder weniger als 33,3 Mikromol/Liter, bevorzugt gleich oder weniger als 29 Mikromol/Liter ist, was indikativ für einen Responder ist, oder mehr als 33,3 Mikromol/Liter ist, was indikativ für einen Nicht-Responder ist.

5. Verfahren nach einem der Ansprüche 1-4, ferner umfassend Bestimmen, ob das Expressionsniveau von Acetyl-L-Carnitin in einer Plasmaprobe des Hepatitis-B-Patienten gleich oder weniger als 3,89 Mikromol/Liter ist, was indikativ für einen Responder ist, oder mehr als 3,89 Mikromol/Liter ist, was indikativ für einen Nicht-Responder ist.

6. Verfahren nach einem der Ansprüche 1-5, ferner umfassend das Bestimmen, ob das Expressionsniveau von Proprionyl-L-Carnitin in einer Plasmaprobe des Hepatitis-B-Patienten gleich oder weniger als 0,43 Mikromol/Liter ist, was indikativ für einen Responder ist, oder mehr als 0,43 Mikromol/Liter ist, was indikativ für einen Nicht-Responder ist.

7. Interferon, optional in Kombination mit einem Hemmer der viralen DNA-Polymerase, zur Verwendung in einem Verfahren zur Behandlung von Hepatitis B, **dadurch gekennzeichnet, dass** ein Hepatitis-B-Patient behandelt wird, der positiv für das G-Allel von SNP rs12356193 getestet wurde.

8. Interferon zur Verwendung in einem Verfahren zur Behandlung von Hepatitis B nach Anspruch 7, wobei ein Hepatitis-B-Patient behandelt wird, der positiv für das G-Allel oder SNP rs12356193, in Kombination mit mindestens einem anderen mit positiven Ausgang verbundenen SNP-Allel getestet wurde, wie in Figur 1 und/oder Figur 2 dargestellt.

9. Verfahren zur Bestimmung, ob ein Individuum eine genetische Veranlagung für eine bessere Aussicht auf einen positiven Ausgang einer Hepatitis-B-Behandlung hat, verglichen mit der mittleren Hepatitis-B-Population, umfassend Bestimmen, ob genomische Nukleinsäure des Individuums mindestens das G-Allel von SNP rs12356193 umfasst.

10. Verfahren nach einem der Ansprüche 1-6 oder 9, ferner umfassend Bestimmen, ob das Individuum einen HBsAg-Basislinienanteil von weniger als 387 IU/ml vor Beginn der Hepatitis-B-Behandlung hat.

## Revendications

1. Procédé pour déterminer si un patient souffrant d'hépatite B a de plus grandes chances d'une issue positive d'un traitement de l'hépatite B, en comparaison de la population moyenne de patients souffrant d'hépatite B, comprenant le fait de déterminer si un échantillon contenant de l'acide nucléique dudit patient souffrant d'hépatite B comprend l'allèle G de SNP rs12356193.

2. Procédé selon la revendication 1, dans lequel ladite issue positive est choisie dans l'ensemble constitué par la perte de HBsAg, la perte de HBsAg avec séroconversion anti-HBs, une réponse virologique durable et une diminution de plus de 1,5 log de HBsAg dans le sang dudit patient à ou avant la semaine 24 de traitement.

3. Procédé selon la revendication 1 ou 2, comprenant le fait de déterminer SNP rs12356193 en association avec au moins un autre SNP tel que représenté sur la figure 1 et/ou la figure 2.

4. Procédé selon l'une quelconque des revendications 1 à 3, comprenant en outre le fait de déterminer si le taux d'expression de la carnitine dans un échantillon de plasma dudit patient souffrant d'hépatite B est égal ou inférieur à 33,3 micromoles/litre, de préférence égal ou inférieur à 29 micromoles/litre, ce qui est indicatif d'un répondeur, ou supérieur à 33,3 micromoles/litre, ce qui est indicatif d'un non-répondeur.

5. Procédé selon l'une quelconque des revendications 1 à 4, comprenant en outre le fait de déterminer si le taux d'expression de l'acétyl-L-carnitine dans un échantillon de plasma dudit patient souffrant d'hépatite B est égal ou inférieur à 3,89 micromoles/litre, ce qui est indicatif d'un répondeur, ou supérieur à 3,89 micromoles/litre, ce qui est indicatif d'un non-répondeur.

6. Procédé selon l'une quelconque des revendications 1 à 5, comprenant en outre le fait de déterminer si le taux d'expression de la propionyl-L-carnitine dans un échantillon de plasma dudit patient souffrant d'hépatite B est égal ou inférieur à 0,43 micromole/litre, ce qui est indicatif d'un répondeur, ou supérieur à 0,43 micromole/litre, ce qui est indicatif d'un non-répondeur.

7. Interféron, en option en association avec un inhibiteur de l'ADN polymérase virale, pour utilisation dans un procédé de traitement de l'hépatite B, **caractérisé en ce qu'**on traite un patient souffrant d'hépatite B qui a été testé positif pour l'allèle G de SNP rs12356193.

8. Interféron pour utilisation dans un procédé de traitement de l'hépatite B selon la revendication 7, dans lequel on traite un patient souffrant d'hépatite B qui a été testé positif pour l'allèle G de SNP rs12356193, en association avec au moins un autre allèle de SNP lié à une issue positive, comme représenté sur la figure 1 et/ou la figure 2.

9. Procédé pour déterminer si un individu a une prédisposition génétique à de plus grandes chances d'une issue positive d'un traitement de l'hépatite B, en comparaison de la population moyenne de patients souffrant d'hépatite B, comprenant le fait de déterminer si l'acide nucléique génomique dudit individu comprend au moins l'allèle G de SNP rs12356193.

10. Procédé selon l'une quelconque des revendications 1 à 6 et 9, comprenant en outre le fait de déterminer si ledit individu a une teneur initiale en HBsAg inférieure à 387 UI/ml avant le début du traitement de l'hépatite B.
